(19) **Europäisches Patentamt / European Patent Office / Office européen des brevets**

(11) **EP 3 404 044 A1**

(12) # EUROPEAN PATENT APPLICATION

(43) Date of publication:
21.11.2018 Bulletin 2018/47

(51) Int Cl.:
*C07K 16/32* (2006.01)　　*C07K 1/18* (2006.01)

(21) Application number: 18161305.0

(22) Date of filing: 22.07.2010

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **24.07.2009 EP 09009640**

(62) Document number(s) of the earlier application(s) in accordance with Art. 76 EPC:
**10740526.8 / 2 456 789**

(71) Applicant: **F. Hoffmann-La Roche AG
4070 Basel (CH)**

(72) Inventors:
• **Hilger, Bernhard
82377 Penzberg (DE)**
• **Burg, Josef
82362 Weilheim (DE)**
• **Kuhne, Wolfgang
82377 Penzberg (DE)**

• **Wallerius, Claus
82377 Penzberg (DE)**
• **Zettl, Frank
82061 Neuried (DE)**
• **Kaiser, Thorsten
82387 Antdorf (DE)**
• **Stiens, Lars
82362 Weilheim (DE)**

(74) Representative: **Vossius & Partner
Patentanwälte Rechtsanwälte mbB
Siebertstrasse 3
81675 München (DE)**

Remarks:
•This application was filed on 12-03-2018 as a divisional application to the application mentioned under INID code 62.
•Claims filed after the date of receipt of the divisional application (Rule 68(4) EPC).

(54) **OPTIMIZING THE PRODUCTION OF ANTIBODIES**

(57) A general method is provided for the production of purified antibodies by separation of an antibody molecule from an antibody variant by chromatographic methods, e.g. to enhance therapeutic efficacy, by for example choosing a specific harvesting time point and/or a specific purification scheme. The current invention thus reports a method for producing an antibody composition comprising an antibody molecule and a variant thereof, comprising the following steps: providing a sample comprising the antibody molecule and a variant thereof, determining the presence of the antibody molecule and/or a variant thereof and/or the ratio of the amount of the antibody molecule or variant thereof to the sum of the amounts of the antibody molecule and the variant thereof, in an aliquot of said sample, determining a subsequent harvesting time point and/or antibody purification scheme on basis of the data obtained before, thereby producing an antibody composition comprising the antibody molecule and a variant thereof.

EP 3 404 044 A1

## Description

### Field of the Invention

The current invention relates to the field of polypeptide production and purification. A general method is provided for the production of purified antibodies by separation of an antibody molecule from an antibody variant by chromatographic methods, e.g. to enhance therapeutic efficacy, by for example choosing a specific harvesting time point and/or a specific purification scheme.

### Background of the Invention

Monoclonal antibodies have great therapeutic potential and play an important role in today's medical portfolio. During the last decade, a significant trend in the pharmaceutical industry has been the development of monoclonal antibodies (mAbs) as therapeutic agents for the treatment of a number of diseases, such as cancers, asthma, arthritis, multiple sclerosis etc. A recent report indicates 376 mAb development programs (from preclinical to market) and monoclonal antibodies currently constitute about 20% of all biopharmaceuticals in clinical trials. Monoclonal antibodies are predominantly manufactured as recombinant proteins in genetically engineered mammalian cell culture. Typically, a stirred tank bioreactor is used for production of the secreted mAb, the upstream process, followed by a downstream process consisting of harvest, chromatographic capture and polishing steps. The drug substance which is typically a liquid is then formulated into drug product.

For human application every pharmaceutical substance has to meet distinct criteria. To ensure the safety of biopharmaceutical agents to humans one or more purification steps have to follow the manufacturing process. The goal of downstream process development is therefore to develop a high-yielding, robust, scalable, and reliable process that results in high-purity product. Typical contaminants in the process include DNA, host cell protein (HCP), viruses, aggregated and fragmented product, and residual media components. Separation of the antibody product from these contaminants requires an orthogonal purification process that utilizes various modes of purification. In general, a mAb purification process involves various combinations of filtration and chromatographic steps. Besides purity, throughput and yield play an important role in determining an appropriate purification process in the pharmaceutical industry. Roughly 40-60% of the production costs for a monoclonal antibody arise from the downstream processes.

According to the International Conference on Harmonization (ICH) guidance document, drug substance heterogeneity defines its quality, and the degree and profile should be monitored and characterized to ensure lot-to-lot consistency. Microheterogeneity of mAbs therefore is a concern for production, especially for downstream processing. Characterization of the product is necessary for determining variants, which may be present with similar properties, and may complicate purification (see e.g. Ahrer, K., and Jungbauer, A., J. Chromatogr. B 841 (2006) 110-122). Microheterogeneity of mAbs for example results from posttranslational modifications, enzymatic modifications, incorrect translation of the target protein, and modifications caused by processing and alteration. Each modification may affect the biological activity or stability of the final product. Thus, rapid, reliable and quantitative analytical methods are needed to resolve several variants of a protein. Chromatographic and electrophoretic methods for example are tools for resolution of protein variants.

The non-enzymatic deamidation of side changes of asparagines and glutamine residues in proteins is often responsible for charge heterogeneity of recombinant proteins. The uncharged side chains of these amino acids are modified to an iso-glutamate and iso-aspartate residue or to a glutamate and aspartate residue. Therefore, an additional charge is introduced to the protein per modification (Aswald, D.W., et al., J. Pharmaceut. Biomed. Anal. 21 (2000) 1129-1136).

For recombinant monoclonal antibodies, deamidation an occur at any stage from inside the cells, after secretion, during purification, during storage, and under different conditions of stress. Deamidation of asparagines has been reported to be involved in the charge heterogeneity of monoclonal antibodies by the observation of more acidic species after incubating antibodies or their separated fractions under basic pH at elevated temperatures (review: Liu, H., et al., J. Pharmac. Sciences 97 (2008) 2426-2447). Deamidation introduces one additional negative charge to antibodies and generates acidic species, which generally results in an earlier elution on cation exchange chromatography.

Other typical modifications of antibodies concern their glycosylation pattern, resulting in glyco-variants, which for example can vary in the course of cell culture or due to the culture conditions (for review, see: Beck, A., et al., Curr. Pharm. Biotechnol. 9 (2008) 482-501).

For the purification of recombinantly produced immunoglobulins often a combination of different column chromatographic steps is employed. Generally an affinity chromatography step is followed by one, two or even more additional separation steps. The final purification step is a so called "polishing step" for the removal of trace impurities and contaminants like aggregated immunoglobulins, residual HCP (host cell protein), DNA (host cell nucleic acid), viruses, or endotoxins.

Tsai, P.K., et al., Pharmac. Res. 10 (1993) 1580-1586, described the isoelectric heterogeneity of an anti-human CD18 monoclonal antibody. The charge heterogeneity was speculated as arising from sequential deamidation of the immunoglobulin heavy chain.

Moorhouse, K.G., et al. (J. Pharmac. Biomed. Anal. 16 (1997) 593-603) discussed the use of HPLC for the analysis of charge heterogeneity of an anti-human CD20 monoclonal antibody.

Kaltenbrunner, O., et al., J. Chrom 639 (1993) 41-49 used a linear pH gradient combined with a salt gradient to separate antibody variants differing in their pI values.

In WO 2006/084111 (Glaxo Group Ltd,) a method is mentioned by which according to the amount of deamidateed polypeptides the harvesting time point is calculated.

Robinson, D,K., et al., Biotech. and Bioeng, 44 (1994) 727-735 disclosed a fed-batch process in which several acidic monoclonal antibody species were produced, wherein the occurrence depended on the culture conditions.

Three mAb variants differing in the presence of lysine residues at the C-terminal of heavy chains have been resolved by cation exchange chromatography using a linear NaCl gradient at neutral pH (Weitzhandler, M., Proteomics 1 (2001) 179-185).

RhuMAb HER2 or Trastuzumab (sold under the trade name Herceptin®) is a recombinant humanized anti-HER2 monoclonal antibody (herein *her2* antibody) used for the treatment of HER2 over-expressed/HER2 gene amplified metastatic breast cancer. Trastuzumab binds specifically to the same epitope of HER2 as the murine anti-HER2 antibody 4D5 described in Hudziak, R.M., et al., Mol. Cell. Biol 9 (1989) 1165-1172. Trastuzumab is a recombinant humanized version of the murine anti-HER2 antibody 4D5, referred to as rhuMAb 4D5 or trastuzumab and has been clinically active in patients with HER2-overexpressing metastatic breast cancers that had received extensive prior anticancer therapy (Baselga, J., et al, J. Clin. Oncol. 14 (1996) 737-744). Trastuzumab and its method of preparation are described in US 5,821,337. The HER family of receptor tyrosine kinases are important mediators of cell growth, differentiation and survival. The receptor family includes four distinct members including epidermal growth factor receptor (EGFR, ErbB1, or HER1), HER2 (ErbB2 or p185$^{neu}$), HER3 (ErbB3) and HER4 (ErbB4 or tyro2).

Monoclonal *her2* antibodies, directed against the gene product of the *her2/neu* gene, were found to exist in seven different variants. Deamidated and other acidic variants of the *her2* antibodies recombinantly produced lead to charge heterogeneity, such that the variants could be separated by cation exchange chromatography using a linear increase in ionic strength for elution. All six minor forms, besides the main peak, could be assigned using a combination of analytical techniques, among others ion exchange chromatography (IEC). Harris R. J. et al. (J. Chromatogr. B 752 (2001), 233-245) reported that the IEC-Peak 3 had a peak area of 73.8 % and represented the active form of *her2* antibodies, which is the most abundant variant. The deamidated and other acidic variants constituted about 25% of all antibodies separated.

EP1075488B1 and EP1308455B9 reported the purification of *her2* antibodies by cation exchange chromatography using wash steps with different conductivities prior to elution (bind-elute modus) at a fourth conductivity. Thereby a composition of *her2* antibodies was obtained wherein the amount of the acidic variants in the composition was reduced. Similarly, WO 2004/024866 (Genentech Inc.) disclosed the purification of her2 antibodies by cation exchange chromatography using in particular gradient wash.

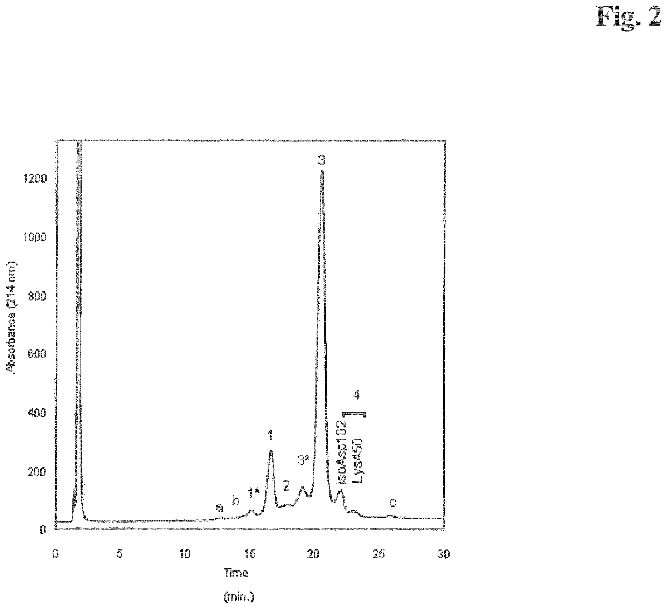

According to the current specification Herceptin® on the market must contain ≥65% of the active form (IEC peak 3/3*) and ≤ 20 % of the inactive form, corresponding to peak 4 in the analytical ion-exchange chromatography profile (see Fig. 2 and Table 1).

**Summary of the Invention**

Thus it is the objective of the current invention to provide another method for the purification of recombinantly produced antibodies and for the enrichment of an antibody molecule relative to an antibody variant thereof.

With the method according to the invention a balance regarding costs and therapeutic efficacy can be obtained. It has been further found that the optimization can be obtained by introducing an in-process-control step during the manufacture of the antibody composition, in which presence of the antibody molecule and/or a variant thereof and/or the ratio of the amount of the antibody molecule or variant thereof and the sum of the amounts of the antibody molecule and the variant thereof, is determined prior to harvest or determination of the purification scheme. With this method, the harvesting time point and purification scheme is adapted to the variant pattern or quality of the antibody source material.

Therefore, the first aspect of the current invention is a method for the production of an antibody composition, comprising an antibody molecule and a variant thereof, comprising the following steps:

a) providing a sample comprising the antibody molecule and a variant thereof,
b) determining the presence of the antibody molecule and a variant thereof and/or the ratio of the amount of the antibody molecule or variant thereof to the sum of the amounts of the antibody molecule and the variant thereof, in an aliquot of said sample

c) determining a subsequent harvesting time point and/or antibody purification scheme on basis of the data obtained in step b),

thereby producing an antibody composition comprising the antibody molecule and a variant thereof.

Another aspect of the current invention is the use of analytical ion exchange chromatography of an aliquot of a sample comprising a polypeptide for determination of a subsequent polypeptide purification scheme of said sample.

## Detailed Description of the Invention

The present invention provides in a first aspect a method for the production of antibody composition enabling the large-scale production of antibodies comprising a high percentage of the antibody molecule in high yield and is highly economic.

Therefore, the first aspect of the current invention is a method for the production of an antibody composition, comprising an antibody molecule and a variant thereof, comprising the following steps:

a) providing a sample comprising the antibody molecule and a variant thereof,
b) determining the presence of the antibody molecule and a variant thereof and/or the ratio of the amount of the antibody molecule and the sum of the amounts of the antibody molecule and the variant thereof,
c) determining a subsequent harvesting time point and/or antibody purification scheme on basis of the data obtained in step b),

thereby producing an antibody composition comprising the antibody molecule and a variant thereof.

In another embodiment comprises the current invention the use of analytical ion exchange chromatography of an aliquot of a sample comprising a polypeptide for determination of a subsequent polypeptide purification scheme of said sample.

The practice of the present invention will employ conventional techniques of molecular biology, microbiology, recombinant DNA techniques, and immunology, which are within the skills of an artisan in the field. Such techniques are explained fully in the literature. See e.g., Sambrook, Fritsch & Maniatis, Molecular Cloning: A Laboratory Manual, Cold Spring Harbor Laboratory Press (1989), Cold Spring Harbor, NY; Glover, D.M. (ed.), DNA Cloning - A Practical Approach, Volumes I and II, IRL Press Limited (1985); Gait, M.J. (ed.), Oligonucleotide Synthesis - A Practical Approach, IRL Press Limited (1984); Hames, B.D. and Higgins, S.J. (eds.), Nucleic acid hybridisation, IRL Press Limited (1984); Freshney, R.I. (ed.) Animal cell culture - a practical approach, IRL Press Limited (1986); Perbal, B., A practical guide to molecular cloning, Wiley Interscience (1984); the series Methods in Enzymology (Academic Press, Inc.); Miller, J.H. and Calos, M.P. (eds.), Gene transfer vectors for mammalian cells, Cold Spring Harbor Laboratory (1987); Methods in Enzymology, Vol. 154 and Vol. 155 (Wu and Grossman, and Wu, eds., respectively); Mayer and Walker (eds.), Immunochemical methods in cell and molecular biology, Academic Press, London (1987); Scopes, R.K., Protein Purification - Principles and Practice, second ed., Springer-Verlag, N.Y. (1987); and Weir, D.M. and Blackwell, C. (eds.), Handbook of Experimental Immunology, Volumes I-IV, Blackwell Scientific Publications (1986).

General chromatographic methods and their use are known to a person skilled in the art. See for example, Heftmann, E. (ed.), Chromatography, fifth ed., Part A: Fundamentals and Techniques, Elsevier Science Publishing Company, New York, (1992); Deyl, Z. (ed.), Advanced Chromatographic and Electromigration Methods in Biosciences, Elsevier Science BV, Amsterdam, The Netherlands (1998); Poole, C. F., and Poole, S. K., Chromatography Today, Elsevier Science Publishing Company, New York (1991); Scopes, R.K., Protein Purification: Principles and Practice, Springer-Verlag, N.Y. (1987); Sambrook, J., et al. (ed), Molecular Cloning: A Laboratory Manual, Second Edition, Cold Spring Harbor Laboratory Press, Cold Spring Harbor, N.Y., (1989); Ausubel, F. M., et al. (eds.), Current Protocols in Molecular Biology, John Wiley & Sons, Inc., New York; or Freitag, R., Chromatographical Techniques in the Downstream Processing of (Recombinant) Proteins, Methods in Biotechnology, Vol. 24: Animal Cell Biotechnology: Methods and Protocols, 2nd ed., Humana Press Inc. (2007), pp. 421-453.

Methods for purifying polypeptides are well established and widespread used. They are employed either alone or in combination. Such methods are, for example, affinity chromatography using microbial-derived proteins (e.g. protein A or protein G affinity chromatography), ion exchange chromatography (e.g. cation exchange (carboxymethyl resins), anion exchange (amino ethyl resins) and mixed-mode exchange chromatography), thiophilic adsorption (e.g. with beta-mercaptoethanol and other SH ligands), hydrophobic interaction or aromatic adsorption chromatography (e.g. with phenyl-sepharose, aza-arenophilic resins, or m-aminophenylboronic acid), metal chelate affinity chromatography (e.g. with Ni(II)- and Cu(II)-affinity material), size exclusion chromatography, and preparative electrophoretic methods (such as gel electrophoresis, capillary electrophoresis) (Vijayalakshmi, M.A., Appl. Biochem. Biotech. 75 (1998) 93-102).

For the purification of recombinantly produced immunoglobulins often a combination of different column chromatographic steps is employed. Generally, a protein A affinity chromatography is followed by one or two additional separation steps. The final purification step is a so called "polishing step" for the removal of trace impurities and contaminants like aggregated immunoglobulins, residual HCP (host cell protein), DNA (host cell nucleic acid), viruses, or endotoxins. For this polishing step often an anion exchange material in a flow-through mode is used.

The following terms, unless otherwise indicated, shall be understood to have the following meanings:

The term "host cells" encompasses plant cells and animal cells. Animal cells encompass invertebrate, non-mammalian vertebrate (e.g., avian, reptile and amphibian) and mammalian cells. Examples of invertebrate cells include the following insect cells: Spodoptera frugiperda (caterpillar), Aedes aegypti (mosquito), Aedes albopictus (mosquito), Drosophila melanogaster (fruitfly), and Bombyx mori (See, e.g., Luckow, V.A. et al., Bio/Technology 6 (1988) 47-55; Miller, D.W., et al., Genetic Engineering, Setlow, J. K. et al. (eds.), Vol. 8, Plenum Publishing (1986), pp. 277-298; and Maeda, S., et al., Nature 315 (1985) 592-594.

The terms "expression" or "expresses" refer to transcription and translation occurring within a host cell. The level of expression of a product gene in a host cell may be determined on the basis of either the amount of corresponding mRNA that is present in the cell or the amount of the polypeptide encoded by the structural gene that is expressed in the cell.

The term "cultivation" as used within this application denotes the entire content of the vessel wherein the fermentation of the host cell, i.e. the production of the he-terologous polypeptide, has been carried out. This comprises the produced he-terologous polypeptide, other proteins and protein fragments present in the medium, host cells, cell fragments, and all constituents supplied with the nutrient medium and produced by the host during the cultivation.

The terms "cell culture medium" and "culture medium" refer to a nutritive solution for the maintenance, growth, propagation, or expansion of cells in an artificial in vitro environment outside of a multicellular organism or tissue. Cell culture medium may be optimized for a specific cell culture use, including, for example, cell culture growth medium which is formulated to promote cellular growth, or cell culture production medium which is formulated to promote recombinant protein production.

The terms "fed batch cell culture" and "fed batch culture," as used herein, refer to a cell culture wherein the cells, preferably mammalian, and culture medium are supplied to the culturing vessel initially and additional culture nutrients are fed, continuously or in discrete increments, to the culture during culturing, with or without periodic cell and/or product harvest before termination of culture. Feeding may be based on the consumption of specific cell culture ingredients.

The term "sample" to be purified herein comprises the polypeptide of interest and one or more contaminants. The composition may for example be "partially purified" (i.e. having been subjected to one or more purification steps, such as Protein A Chromatography) or for example may be obtained directly from a host cell or organism producing the polypeptide (e.g. the composition may comprise harvested cell culture fluid).

The term "contaminant" refers to any foreign or undesirable molecule that is present in a solution such as a load fluid. A contaminant can be a biological macromolecule such as a DNA, an RNA, or a protein, other than the protein of interest being purified, that is also present in a sample of the protein of interest being purified. Contaminants include, for example, undesirable protein variants, such as aggregated proteins, misfolded proteins, underdisulfide-bonded proteins, high molecular weight species; other proteins from host cells that secrete the protein being purified, host cell DNA, components from the cell culture medium, molecules that are part of an absorbent set for affinity chromatography that leach into a sample during prior purification steps, for example, Protein A; an endotoxin; a nucleic acid; a virus; or a fragment of any of the foregoing.

The term "chromatography material" as used within this application denotes a solid material comprising a bulk core material to which chromatographical functional groups are attached, preferably by covalent bonds. The bulk core material is understood to be not involved in the chromatography process, i.e. the interaction between the polypeptide to be separated and the chromatographical functional groups of the chromatography material. It is merely providing a three dimensional framework to which the chromatographical functional groups are attached and which ensures that the solution containing the substance to be separated can access the chromatographical functional group. Preferably said bulk core material is a solid phase. Thus, preferably said "chromatography material" is a solid phase to which chromatographical functional groups are attached, preferably by covalent bonds. Preferably said "chromatographical functional group" is an ionizable hydrophobic group, or a hydrophobic group, or a complex group in which different chromatographical functional groups are combined in order to bind only a certain type of polypeptide, or a covalently bound charged group.

A "solid phase" denotes a non-fluid substance, and includes particles (including microparticles and beads) made from materials such as polymer, metal (paramagnetic, ferromagnetic particles), glass, and ceramic; gel substances such as silica, alumina, and polymer gels; zeolites and other porous substances. A solid phase may be a stationary component, such as a packed chromatography column, or may be a non-stationary component, such as beads and microparticles. Such particles include polymer particles such as polystyrene and poly(methylmethacrylate); gold particles such as gold nanoparticles and gold colloids; and ceramic particles such as silica, glass, and metal oxide particles. See for example Martin, C.R., et al., Analytical Chemistry-News & Features (1998) 322A-327A.

The terms "hydrophobic charge induction chromatography" or "HCIC", which can be used interchangeably within this application, denote a chromatography method which employs a "hydrophobic charge induction chromatography material". A "hydrophobic charge induction chromatography material" is a chromatography material which comprises chromatographic function groups which can in one pH range form hydrophobic bonds to the substance to be separated and which are charged either positively or negatively in other pH ranges, i.e. HCIC uses ionizable hydrophobic groups a chromatographic functional group. Generally the polypeptide is bound to the hydrophobic charge induction material under neutral pH conditions and recovered afterwards by the generation of charge repulsion by a change of the pH value. An exemplary "hydrophobic charge induction chromatography materials" is BioSepra MEP or HEA Hypercell (Pall Corp., USA).

The terms "hydrophobic interaction chromatography" or "HIC", which can be used interchangeably within this application, denote a chromatography method in which a "hydrophobic interaction chromatography material" is employed. A "hydrophobic interaction chromatography material" is a chromatography material to which hydrophobic groups, such as butyl-, octyl-, or phenyl-groups, are bound as chromatographic functional groups. The polypeptides are separated depending on the hydrophobicity of their surface exposed amino acid side chains, which can interact with the hydrophobic groups of the hydrophobic interaction chromatography material. The interactions between polypeptides and the chromatography material can be influenced by temperature, solvent, and ionic strength of the solvent. A temperature increase e.g. supports the interaction between the polypeptide and the hydrophobic interaction chromatography material as the motion of the amino acid side chains increases and hydrophobic amino acid side chains buried inside the polypeptide at lower temperatures become accessible. Also is the hydrophobic interaction promoted by kosmotropic salts and decreased by chaotropic salts. "Hydrophobic interaction chromatography materials are e.g. Phenylsepharose CL-4B, 6 FF, HP, Phenyl Superose, Octylsepharose CL-4B, 4 FF, and Butylsepharose 4 FF (all available from GE Healthcare Life Sciences, Germany), which are obtained via glycidyl-ether coupling to the bulk material.

The term "affinity chromatography" as used within this application denotes a chromatography method which employs an "affinity chromatography material". In an affinity chromatography the polypeptides are separated based on their biological activity or chemical structure depending of the formation of electrostatic interactions, hydrophobic bonds, and/or hydrogen bond formation to the chromatographic functional group. To recover the specifically bound polypeptide from the affinity chromatography material either a competitor ligand is added or the chromatography conditions, such as pH value, polarity or ionic strength of the buffer are changed. An "affinity chromatography material" is a chromatography material which comprises a complex chromatographic functional group in which different single chromatographic functional groups are combined in order to bind only a certain type of polypeptide. This chromatography material specifically binds a certain type of polypeptide depending on the specificity of its chromatographic functional group. Exemplary "affinity chromatographic materials" are a "metal chelating chromatography material" such as Ni(II)-NTA or Cu(II)-NTA containing materials, for the binding of fusion polypeptides containing a hexahistidine tag or polypeptides with a multitude of surface exposed histidine, cysteine, and/or tryptophan residues, or an "antibody binding chromatography material" such as protein A, or antigens, or an "enzyme binding chromatography material" such as chromatography materials comprising enzyme substrate analogues, enzyme cofactors, or enzyme inhibitors as chromatographic functional group, or a "lectin binding chromatography material" such as chromatography materials comprising polysaccharides, cell surface receptors, glycoproteins, or intact cells as chromatographic functional group.

When used herein, the term "Protein A" encompasses Protein A recovered from a native source thereof, Protein A produced synthetically (e.g. by peptide synthesis or by recombinant techniques), and variants thereof which retain the ability to bind proteins which have a CH2/CH3 region. Protein A can be purchased commercially from GE Healthcare Life Sciences, Germany, for example.

"Protein A affinity chromatography" refers to the separation or purification of substances and/or particles using protein A, where the protein A is generally immobilized on a solid phase. A protein comprising a CH2/Ch3 region may be reversibly bound to or adsorbed by the protein A. Examples of protein A affinity chromatography columns for use in protein A affinity chromatography herein include protein A immobilized onto a controlled pore glass backbone, protein A immobilized on a polystyrene solid phase, e.g. the POROS 50A(TM) column (Applied BioSystems Inc.); or protein A immobilized on an agarose solid phase, for instance the rPROTEIN A SEPHAROSE FAST FLOW(TM) or MABSELECT(TM) columns (GE Healthcare Life Sciences, Germany).

The term "metal chelate chromatography" as used within this application denotes a chromatography method which employs a "metal chelate chromatography material". Metal chelate chromatography is based on the formation of chelates between a metal ion, such as Cu(II), Ni(II) or Zn(II), which is bound to a bulk material as chromatographic functional groups, and electron donor groups of surface exposed amino acid side chains of polypeptides, especially with imidazole containing side chains and thiol group containing side chains. The chelate is formed at pH values at which those side chains are at least partly not protonated. The bound polypeptide is recovered from the chromatography material by a change in the pH value, i.e. by protonation. Exemplary "metal chelating chromatography materials" are HiTrap Chelating HP (GE Healthcare Life Sciences, Germany), or Fractogel EMD (EMD Chemicals Inc, USA).

The term "ion exchange chromatography" as used within this application denotes a chromatography method which employs an "ion exchange chromatography material". The term "ion exchange chromatography material" encompasses depending whether a cation is exchanged in a "cation exchange chromatography" a "cation exchange chromatography material" or an anion is exchanged in an "anion exchange chromatography" an "anion exchange chromatography material". The term "ion exchange chromatography material" as used within this application denotes an immobile high molecular weight solid phase that carries covalently bound charged groups as chromatographic functional groups. For overall charge neutrality not covalently bound counter ions are associated therewith. The "ion exchange chromatography material" has the ability to exchange its not covalently bound counter ions for similarly charged ions of the surrounding solution. Depending on the charge of its exchangeable counter ions the "ion exchange chromatography material" is referred to as "cation exchange chromatography material" or as "anion exchange chromatography material". Further depending on the nature of the charged group the "ion exchange chromatography material" is referred to as e.g. in the case of cation exchange chromatography materials with sulfonic acid groups (S), or carboxymethyl groups (CM). Depending on the chemical nature of the charged group the "ion exchange chromatography material" can additionally be classified as strong or weak ion exchange chromatography material, depending on the strength of the covalently bound charged substituent. For example, strong cation exchange chromatography materials have a sulfonic acid group as chromatographic functional group and weak cation exchange chromatography materials have a carboxylic acid group as chromatographic functional group. "Cation exchange chromatography materials", for example, are available under different names from a multitude of companies such as e.g. Bio-Rex, Macro-Prep CM (available from BioRad Laboratories, Hercules, CA, USA), weak cation exchanger WCX 2 (available from Ciphergen, Fremont, CA, USA), Dowex® MAC-3 (available from Dow chemical company - liquid separations, Midland, MI, USA), Mustang C (available from Pall Corporation, East Hills, NY, USA), Cellulose CM-23, CM-32, CM-52, hyper-D, and partisphere (available from Whatman pic, Brentford, UK), Amberlite® IRC 76, IRC 747, IRC 748, GT 73 (available from Tosoh Bioscience GmbH, Stuttgart, Germany), CM 1500, CM 3000 (available from BioChrom Labs, Terre Haute, IN, USA), and CM-Sepharose™ Fast Flow (available from GE Healthcare, Life Sciences, Germany). Commercially available cation exchange resins further include carboxy-methyl-cellulose, Bakerbond ABX™, sulphopropyl (SP) immobilized on agarose (e.g. SP-Sepharose Fast Flow™ or SP-Sepharose High Performance™, available from GE Healthcare - Amersham Biosciences Europe GmbH, Freiburg, Germany) and sulphonyl immobilized on agarose (e.g. S-Sepharose Fast Flow ™ available from GE Healthcare, Life Sciences, Germany).

The term "hydroxylapatite chromatography" as used within this application denotes a chromatography method that employs a certain form of calcium phosphate as chromatography material. Exemplary hydroxylapatite chromatography materials are Bio-Gel HT, Bio-Gel HTP, Macro-Prep Ceramic (available from BioRad Laboratories), Hydroxylapatite Type I, Type II, HA Ultrogel (Sigma Aldrich Chemical Corp., USA), Hydroxylapatite Fast Flow and High Resolution (Calbiochem), or TSK Gel HA-1000 (Tosoh Haas Corp., USA).

The term "buffered" as used within this application denotes a solution in which changes of pH due to the addition or release of acidic or basic substances is leveled by a buffer substance. Any buffer substance resulting in such an effect can be used. Preferably pharmaceutically acceptable buffer substances are used, such as e.g. phosphoric acid or salts thereof, acetic acid or salts thereof, citric acid or salts thereof, morpholine or salts thereof, 2-(N-morpholino) ethanesulfonic acid or salts thereof, histidine or salts thereof, glycine or salts thereof, or Tris (hydroxymethyl) aminomethane (TRIS) or salts thereof. Especially preferred are phosphoric acid or salts thereof, or acetic acid or salts thereof, or citric acid or salts thereof, or histidine or salts thereof. Optionally the buffered solution may comprise an additional salt, such as e.g. sodium chloride, sodium sulphate, potassium chloride, potassium sulfate, sodium citrate, or potassium citrate.

The term "membrane" as used within this application denotes both a microporous or macroporous membrane. The membrane itself is composed of a polymeric material such as, e.g. polyethylene, polypropylene, ethylene vinyl acetate copolymers, polytetrafluoroethylene, polycarbonate, poly vinyl chloride, polyamides (nylon, e.g. Zetapore™, $N_{66}$ Posidyne™), polyesters, cellulose acetate, regenerated cellulose, cellulose composites, polysulphones, polyethersulphones, polyarylsulphones, polyphenylsulphones, polyacrylonitrile, polyvinylidene fluoride, non-woven and woven fabrics (e.g. Tyvek®), fibrous material, or of inorganic material such as zeolithe, $SiO_2$, $Al_2O_3$, $TiO_2$, or hydroxylapatite.

The "loading buffer" is that which is used to load the composition comprising the polypeptide molecule of interest and one or more contaminants onto the ion exchange resin. The loading buffer has a conductivity and/or pH such that the polypeptide molecule of interest (and generally one or more contaminants) is/are bound to the ion exchange resin.

The term "wash buffer" refers to a buffer which is used to wash or re-equilibrate the chromatography material, e.g. the ion exchange resin, prior to eluting the polypeptide molecule of interest. The wash buffer and loading buffer may be the same, but this is not required.

The "elution buffer" refers to the buffer which is used to elute the polypeptide of interest from the solid phase. For example the conductivity and/or pH of the elution buffer are adapted such that the polypeptide of interest can be eluted from the chromatography material, e.g. the ion exchange resin.

The term "regeneration buffer" refers to a buffer that may be used to regenerate the chromatography material, e.g. the ion exchange or Protein A resin such that it can be re-used.

The term "conductivity" refers to the ability of an aqueous solution to conduct an electric current between two electrodes. The unit of measurement for conductivity is mS/cm, and can be measured using a conductivity meter. The conductivity of a solution may be altered by changing the amount of ions in therein. For example, the amount of a buffering agent and/or amount of a salt (e.g. NaCl) in the solution may be altered in order to achieve the desired conductivity.

By "purifying" an antibody or a polypeptide from a solution comprising the polypeptide and one or more contaminants is meant increasing the degree of purity of the antibody or polypeptide in the solution by removing at least one contaminant from the composition.

The "pI" or "isoelectric point" of a polypeptide refer to the pH at which a protein carries no net charge. Below the isoelectric point proteins carry a net positive charge, above it a net negative charge. The isoelectric point is of significance in protein purification and can for example be determined with isoelectric focusing or with computer programs. Further, analytical ion exchange chromatography could resolve protein variants with very similar pI values, i.e. differing for example only by 0.1 pI unit.

By "binding" a molecule to a chromatography material, e.g. an ion exchange material, is meant exposing the molecule to the ion exchange material under appropriate conditions (pH/conductivity) such that the molecule is reversibly immobilized in or on the chromatography material, e.g. an ion exchange material by virtue of ionic interactions between the molecule and a charged group or charged groups of the ion exchange material.

By "washing" material is meant passing an appropriate buffer through or over the chromatography material, e.g. the ion exchange material.

To "elute" a molecule denotes the act of separating one substance (e.g. polypeptide or contaminant) from another (e.g. an antibody) by means of a solvent, e.g. from a chromatography material.

The term "bind-and-elute mode" and grammatical equivalents thereof as used in the current invention denotes an operation mode of a chromatography method, in which a solution containing a substance of interest is brought in contact with a stationary phase, preferably a solid phase, whereby the substance of interest binds to the stationary phase. As a result the substance of interest is retained on the stationary phase whereas substances not of interest are removed with the flow-through or the supernatant. The substance of interest is afterwards eluted from the stationary phase in a second step and thereby recovered from the stationary phase with an elution solution. This does not necessarily denote that 100% of the substances not of interest are removed but essentially 100% of the substances not of interest are removed, i.e. at least 50% of the substances not of interest are removed, preferably at least 75% of the substances not of interest are removed, preferably at least 90% of the substances not of interest are removed, preferably more than 95% of the substances not of interest are removed.

The term "flow-through mode" and grammatical equivalents thereof as used within the current invention denotes an operation mode of a chromatography method, in which a solution containing a substance of interest is brought in contact with a stationary phase, preferably a solid phase, whereby the substance of interest does not bind to that stationary phase. As a result the substance of interest is obtained either in the flow-through or the supernatant. Substances not of interest, which were also present in the solution, bind to the stationary phase and are removed from the solution. This does not necessarily denote that 100% of the substances not of interest are removed from the solution but essentially 100% of the substances not of interest are removed, i.e. at least 50% of the substances not of interest are removed from the solution, preferably at least 75% of the substances not of interest are removed the from solution, preferably at least 90% of the substances not of interest are removed from the solution, preferably more than 95% of the substances not of interest are removed from the solution.

The terms "gradient elution", "gradient elution mode", "continuous elution" and "continuous elution method", which are used interchangeably within this application, denote herein a chromatography method wherein e.g. the amount of a substance causing elution, i.e. the dissolution of a bound substance from a chromatography material, is raised or lowered continuously, i.e. the amount is changed by a sequence of small steps each not bigger than a change of 2 %, preferably of 1%, of the amount of the substance causing elution. In this "gradient elution" one or more conditions, for example the pH, the ionic strength, amount of a salt, and/or the flow of a chromatography method, may be changed linearly, or changed exponentially, or changed asymptotically. Preferably the change is linear. These linear changes can be separated by stationary phases. Moreover, the steepness of the linear changes may vary during elution. Step elution may follow gradient elution in a specific chromatography step for elution of the bound molecule from a specific column, as described below.

The terms "step elution", "step elution mode" and "step elution method", which are used interchangeably within this application, denote a chromatography method wherein e.g. the amount of a substance causing elution, i.e. the dissolution of a bound substance from a chromatography material, is raised or lowered at once, i.e. directly from one value/level to the next value/level. In this "step elution" one or more conditions, for example the pH, the ionic strength, amount of a salt, and/or the flow of a chromatography method, is/are changed all at once from a first, e.g. starting, value to a second, e.g. final, value. The change in the step is bigger than a change of 5%, preferably of 10%, of the amount of the substance causing elution. "Step elution" denotes that the conditions are changed incrementally, i.e. stepwise, in contrast to a linear change. After each increase the conditions are maintained till the next step in the elution method.

The term "step elution only" refers to elution from a certain chromatography column by using only step elution for eluting a polypeptide and not gradient elution in the respective chromatography step.

A "single step" denotes a process wherein one or more conditions, for example the pH, the ionic strength, amount of a salt, and/or the flow of a chromatography, is/are changed all at once from a starting value to a final value, i.e. the conditions are changed incrementally, i.e. stepwise, in contrast to a linear change.

The term "applying to" and grammatical equivalents thereof as used within this application denotes a partial step of a purification method in which a solution containing a substance of interest to be purified is brought in contact with a stationary phase. This denotes that a) the solution is added to a chromatographic device in which the stationary phase is located, or b) that a stationary phase is added to the solution. In case a) the solution containing the substance of interest to be purified passes through the stationary phase allowing for an interaction between the stationary phase and the substances in solution. Depending on the conditions, such as e.g. pH, conductivity, salt amount, temperature, and/or flow rate, some substances of the solution are bound to the stationary phase and thus are removed from the solution. Other substances remain in solution. The substances remaining in solution can be found in the flow-through. The "flow-through" denotes the solution obtained after the passage of the chromatographic device. Preferably the chromatographic device is a column, or a cassette. The substance of interest not bound to the stationary phase can be recovered from the flow-though by methods familiar to a person of skill in the art, such as e.g. precipitation, salting out, ultrafiltration, diafiltration, lyophilization, affinity chromatography, or solvent volume reduction to obtain a concentrated solution. In case b) the stationary phase is added, e.g. as a powder, to the solution containing the substance of interest to be purified allowing for an interaction between the stationary phase and the substances in solution. After the interaction the stationary phase in removed, e.g. by filtration, and the substance of interest not bound to the stationary phase is obtained in the supernatant.

The term "does not bind to" and grammatical equivalents thereof as used within this application denotes that a substance of interest, e.g. an immunoglobulin, remains in solution when brought in contact with a stationary phase, e.g. an ion exchange material. This does not necessarily denote that 100% of the substance of interest remains in solution but essentially 100% of the substance of interest remains in solution, i.e. at least 50% of the substance of interest remains in solution, preferably at least 65% of the substance of interest remains in solution, preferably at least 80% of the substance of interest remains in solution, preferably at least 90% of the substance of interest remains in solution, preferably more than 95% of the substance of interest remains in solution.

The term "In-Process-Control Parameter" (IPC) is a parameter used for monitoring a reaction process applied to process validation. IPCs could for example be monitored during cultivation of cells expressing an antibody. By measuring or determining the "In-process-control Parameter" a value is obtained triggering a specific action on the reaction process. Examples for "IPCs" are for example oxygen and glucose levels, pH and $CO_2$ values, during fermentation of an antibody. The action triggered by measuring these parameters could for example be a feed-back control on the oxygen and glucose supply. Other IPCs could yield information on the antibody to be produced, e.g. the amount in the cultivation medium, the quality, e.g. the percentage of active and inactive variants, or the content of specific glycovariants. For determining a specific IPC a sample could for example be withdrawn from the medium or the IPC could be determined online during fermentation.

A "polypeptide" is a polymer consisting of amino acids joined by peptide bonds, whether produced naturally or synthetically. Polypeptides of less than about 20 amino acid residues may be referred to as "peptides", whereas molecules consisting of two or more polypeptides or comprising one polypeptide of more than 100 amino acid residues may be referred to as "proteins". A polypeptide may also comprise non-amino acid components, such as carbohydrate groups, metal ions, or carboxylic acid esters. The non-amino acid components may be added by the cell, in which the polypeptide is expressed, and may vary with the type of cell. Polypeptides are defined herein in terms of their amino acid backbone structure or the nucleic acid encoding the same. Additions such as carbohydrate groups are generally not specified, but may be present nonetheless.

The term "recombinant polypeptide" refers to a polypeptide which has been produced in a host cell which has been transformed or transfected with nucleic acid encoding the polypeptide, or produces the polypeptide as a result of homologous recombination.

The term "heterologous DNA" or "heterologous polypeptide" refers to a DNA molecule or a polypeptide, or a population of DNA molecules or a population of polypeptides that do not exist naturally within a given cell. DNA molecules heterologous to a particular cell may contain DNA derived from the cell's species (i.e. endogenous DNA) so long as that cell's DNA is combined with non-cell's DNA (i.e. exogenous DNA). For example, a DNA molecule containing a non-cell's DNA segment encoding a polypeptide operably linked to a cell's DNA segment comprising a promoter is considered to be a heterologous DNA molecule. Conversely, a heterologous DNA molecule can comprise an endogenous structural gene operably linked with an exogenous promoter. A peptide or polypeptide encoded by a non-cell's DNA molecule is a "heterologous" peptide or polypeptide.

The term "antibody" refers to any immunoglobulin or fragment thereof, and encompasses any polypeptide comprising an antigen-binding site. The term includes but is not limited to, polyclonal, monoclonal, monospecific, polyspecific, non-specific, humanized, human, single-chain, chimeric, synthetic, recombinant, hybrid, mutated, grafted, bispecific, trispecifc and in vitro generated antibodies. Antibody fragments include Fab, F(ab')$_2$, Fv, scFv, Fd, dAb, which may retain antigen-binding function. Typically, such fragments include an antigen-binding domain.

The term "amount" as used herein corresponds to the quantity of a polypeptide or antibody. The amount can be expressed in relative units, e.g. area under peak in chromatogram, or for example in $\mu$g.

An "antibody molecule" as used herein refers to the antibody of interest, which for example could be the most active form in comparison to the variants thereof. The variants and the antibody molecule are expressed from the same sequence.

An "active antibody or active variant" as used herein refers to an antibody with a high biological potency, i.e. the activity of the "active variant" is between 70 and 150% of the average activity of all antibody variants (antibody variants are expressed from the same antibody sequence). For example the Herceptin variant which carries no deamidation or isomerization of its asparagine residues, corresponding to the main peak, peak 3/3*, in the ion exchange chromatogram (see Fig. 2 and Table 1) has the highest biological potency in comparison to the other variants separated.

An "acidic variant" of an antibody molecule is a variant of an antibody molecule of interest which is more acidic (e.g. determined by isoelectric focusing or ion exchange chromatography) than the antibody molecule of interest. An example of an acidic variant is a deamidated variant. Also included are all variants which elute in the acidic region, in comparison to the main peak, during ion exchange chromatography.

A "deamidated" variant of a polypeptide molecule is a polypeptide wherein for example one or more asparagine residue(s) of the original polypeptide have been converted to aspartate, i.e. the neutral amide side chain has been converted to a residue with an overall acidic character. A deamidated *her2* variant for example is a *her2* antibody variant with a conversion of asparagine to aspartate at amino acid position 30, e.g. corresponding to peak 1 in the ion exchange chromatogram (Fig. 2, Table 1).

A "basic variant" of an antibody molecule is a variant of an antibody molecule which is more basic (e.g. determined by isoelectric focusing or ion exchange chromatography) than the antibody molecule of interest. Here, also variants are included, which only differ for example by an isomerization of asparagine, which theoretically should not alter the overall theoretical charge of the antibody, but which might result in a conformation of the antibody such that the antibody elutes in the more basic region in comparison to the unmodified antibody, during ion exchange chromatography. This is for example the case with the herceptin variant resolved in peak 4 (Fig. 2, Table 1).

The term glyco-variant refers to an antibody variant which is characterized by a different glycosylation pattern in comparison to the antibody molecule. That means that the type and distribution of polysaccharides attached to the antibody (the glycans) is different among the glyco-variants. A glyco-variant of an antibody molecule may also fall into the category basic or acidic variant if their pI value differs from the antibody molecule.

All antibody variants are expressed from the same sequence as the antibody molecule and thus are for example post-translationally modified, either *in vivo* or *in vitro.*

The term "threshold ratio" as used herein refers to the ratio of the amount of the antibody molecule or variant thereof to the sum of the amounts of the antibody molecule and the variant thereof at which a specific decision is drawn. For example the threshold ratio could be decisive for the purification scheme or harvesting time point during fermentation. Or is decisive for the type of cation exchange chromatograph performed, i.e. below the threshold ratio elution is performed differently from the elution performed if the ratio is higher than the threshold ratio (gradient *versus* step elution for example).

The term "ratio of the amount of the antibody molecule or variant therof to the sum of the amounts of the antibody molecule and the variant thereof" refers to a ratio which corresponds to the quotient of the amount of the antibody molecule or variant thereof in a certain volume to the amount of the antibody molecule and the variants thereof in the same volume. Thus, the amount of the antibody molecule or variant thereof is set in relation to the sum of the amount of the antibody molecule and the total amounts of the variants thereof. Thus, for each antibody variant and the antibody molecule in an antibody containing solution a certain ratio can attributed and the sum of all these ratios must be 100%. Such ratios can for example be determined from ion exchange chromatograms, as shown in Fig. 2, wherein the UV absorption is plotted, or after determining the antibody content via protein measurements. No absolute protein measurements are necessary for determination of the ratio, since it is only a ratio and not an absolute value, but only UV absorption, corresponding to a certain protein amount, could be sufficient for calculating the ratio. For example, the peak areas under a chromatogram, can be used for determination of the ratio of the amount of an antibody molecule or variant thereof to the sum of the antibody molecule and variant thereof. The sum of the area of all peaks (antibody molecule and variants) would then correspond the denominator and the area under single peaks to the respective numerators.

Unless indicated otherwise, the term *"her2"* when used herein refers to human *her2* protein and *"her2"* refers to human *her2* gene. The human *her2* gene and *her2* protein are described in Semba et al., PNAS (USA) 82:6497-6501 (1985) and Yamamoto et al. Nature 319:230-234 (1986) (Genbank accession number X03363), for example.

The term "drug product" as used herein refers to the purified antibody in the formulation buffer, which is used for the treatment of patients. The drug product contains a minimum level of the active antibody variant and maximum level of the inactive variant. These values are evaluated during the development and submitted to the Health Authorities.

"Treatment" refers to both therapeutic treatment and prophylactic or preventative measures. Those in need of treatment include those already with the disorder as well as those in which the disorder is to be prevented.

A "disorder" is any condition that would benefit from treatment with the polypeptide purified as described herein. This includes chronic and acute disorders or diseases including those pathological conditions which predispose the mammal to the disorder in question.

During fermentation of an antibody not only the antibody molecule, that is the antibody of interest, but also variants of that antibody are produced. These variants could have similar activities in comparison to the antibody molecule but could also be less active, for example during treatment in a patient and might be unwanted in the bulk drug product. One example could be a deamidated antibody variant, which might be more susceptible to in vivo degradation. The method according to the invention enables the production of an antibody molecule at high yield and high purity with regard to unwanted variants. The method according to the invention is based on the surprising finding that the antibody composition with regard to the variant distribution, which can be expressed for example as a ratio of the amount of the antibody molecule to sum of the amounts of the antibody molecule and the variant thereof, can be shifted to the desired direction by adapting the harvesting time point of the antibody and/or subsequent antibody purification scheme.

Thus, the current invention provides in a first aspect a method for the production of an antibody composition, comprising an antibody molecule and a variant thereof, comprising the following steps:

a) providing a sample comprising the antibody molecule and a variant thereof,
b) determining the presence of the antibody molecule and a variant thereof and/or the ratio of the amount of the antibody molecule or variant thereof to the sum of the amounts of the antibody molecule and the variant thereof, in an aliquot of said sample,
c) determining a subsequent harvesting time point and/or antibody purification scheme on basis of the data obtained in step b),

thereby producing an antibody composition comprising the antibody molecule and a variant thereof.

In a preferred aspect of the current invention the sample is a cell culture medium comprising an antibody molecule and a variant thereof free from cells and cellular debris or is an eluate of a Protein A affinity chromatography step. In still further aspects of the current invention the sample comprising the antibody molecule and the variant thereof is an eluate obtained from other columns used for antibody separation, like anion exchange columns, hydrophobic interaction columns, hydroxyapatite chromatography columns etc.

In certain aspects of the current invention, the variant of the antibody molecule is an acidic variant, a basic variant, a glyco-variant, or a variant with a different binding affinity to a defined antigen in comparison to the antibody molecule.

In still another preferred embodiment, the variant of the antibody molecule is a less active variant than the antibody molecule itself. Activity could for example be measured in "in vitro" assays (e.g. efficacy to neutralize an antigen) or in an animal model for example.

In another preferred embodiment, the pI of the antibody molecule differs by 0.1 to 0.5 pI units from the pI of the antibody molecule. PI values are determined with theoretical software programs, if for example the sequence of the protein to be analyzed is known, or by methods known by the persons skilled in the art, like isoelectric focusing, for example.

In a certain aspect of the invention, providing a sample comprising the medium, the cell, the antibody molecule and a variant thereof comprises the following steps:

i) providing a cell containing a nucleic acid molecule comprising a nucleic acid sequence encoding said antibody molecule,
ii) cultivating said cell in a medium for 4-28 days,
iii) obtaining a sample from the medium,

In still further aspects of the invention cultivating of the cells in a medium in step ii) is at least for 4-28 days, preferably for 4-18 days and most preferred for 4-16 days.

In still further aspects of the invention cultivating of the cell in a medium in step ii) is performed until a specific antibody concentration is obtained, at least 200 mg, preferably at least 800 mg/l, more preferably at least 1000 mg/ml and most preferred at least 1500 mg/ml. These concentrations depend among others on the kind of antibody expressed. In the case of Herceptin, an antibody concentration of at least 200 mg/l is preferred.

In a preferred aspect of the current invention, the presence of the antibody molecule and/or variant thereof and the ratio of the amount of the antibody molecule or variant thereof and the sum of the antibody molecule and the variant thereof is determined by ion exchange chromatography, e.g. by analytical ion exchange chromatography. This technique is generally known by the skilled person in the art and involves for example cation exchange chromatography using gradient elution. For calculation of the relative amounts of the antibody molecule and the variant thereof the area under the peaks is determined for the respective peaks resolved (for details of a possible method, see the Examples below). Determination of the presence of the antibody molecule and/or variant thereof is the mere determination whether the antibody molecule and/or variant thereof is contained in a specific sample or not. The mere observation that a specific variant is contained, irrespective of their relative amount, could be decisive for the harvesting time point or purification scheme.

In further embodiments of the current invention, the percentage of the antibody molecule, i.e. the antibody molecule of interest, or variant thereof, is determined by other suitable methods known by the skilled person, e.g. by isoelectric focusing (see in this context also the review of Ahrer and Jungbauer: J. of Chromatog. 2006, Vol. 841, pages 110-122.

In further aspects of the current invention the presence and/or amount of glyco-variants is determined, e.g. by MALDI-TOF analysis or other methods known by the skilled person in the art.

In still further aspects of the current invention the amount of the most active *her2* antibody variant, corresponding to peak 3/3* in the ion exchange chromatogram, relative to the sum of the most active variant and the other variants thereof, is determined, see for example Fig. 2 and Table 1 (cf. Harris, R. J., J. Chromatogr. B 752 (2001) 233-245).

In another embodiment of the current invention, the presence or ratio of the antibody molecule and/or variant thereof is determined daily, every second day, or in other defined increments, after for example 3, 5, or 7 d of fermentation or daily or every second d after a certain antibody concentration in the fermentation medium, 0.2 to 1.5 mg/ml, is reached. In still further embodiments of the current invention, the relative amount of the active antibody is determined only once during the fermentation after a certain length of fermentation, preferably after 10, 11 or 12 d of fermentation for Herceptin.

In a certain embodiment of the method of the current invention, the total antibody amount (sum of the amount of the antibody molecule and all variants in the sample) at a certain fermentation day is determined using methods known by the skilled person, like for example protein measurements and ELISA. In another aspect of the current invention, the total antibody amount at a certain time point is calculated on basis of the time course of the total antibody amount known for previous similar fermentation runs. In the Examples below an exemplary method is described for the determination of the *her2* amount in a sample.

In a preferred aspect of the current invention, the antibody molecule and variant thereof is purified by cation exchange chromatography, optionally after performing an affinity chromatography step. The elution modes used, however, depend on the purity of the source material, i.e. on the ratio of the amount of the antibody molecule to the sum of the amounts of the antibody molecule and a variant thereof. According to a preferred aspect of the current invention, elution of the antibody from the cation exchange column is performed

i) by a gradual increase of conductivity and/or pH of the buffer applied to the cation exchange column, and

ii) second, by a step wise increase of conductivity and/or pH of the elution buffer applied to the cation exchange column, if the ratio of the amount of the antibody molecule to the sum of the amounts of the antibody molecule and a variant thereof determined in step a1) is below a threshold ratio, or

with a step wise increase of conductivity and/or pH without a gradual increase of conductivity and/or pH of the elution buffer applied to the column, if the ratio of the amount of the antibody molecule to the sum of the amounts of the antibody molecule and a variant thereof determined in step a1) is above a threshold ratio.

This threshold ratio in the context of the current invention can be determined as follows: The aim is a formulated therapeutic antibody drug (bulk drug product) with a predetermined minimum level of the antibody molecule of interest and a predetermined maximum level of a variant thereof, set out in the specification of that antibody. These levels are for example relevant for the Regulatory Authorities. The variant might for example be less active in vivo is unwanted for other reasons. This threshold ratio now determines the harvesting time point and purification scheme, in particular the mode of elution during cation exchange chromatography. For example, below the threshold ratio, the antibody is purified by gradient elution, followed by step elution, in the cation exchange chromatography step in order to obtain a sufficiently high purity of the antibody molecule and yield, and above that ratio the antibody will be purified by step elution only in order to obtain high yields and still antibodies fulfilling the safety criteria. Thus, the threshold is set such, that antibodies, which meet the Regulatory Authority demands, are obtainable irrespective of the nature of the source material, and that highest possible yields can be obtained. In another preferred embodiment, the threshold ratio determines the harvesting time point, such that for example harvesting is only performed if values above the threshold value are reached, see below, in order to always use the step elution only mode in cation exchange chromatography. The background here is that during fermentation, specific antibody variants might occur and increase in their relative amounts whereas the amount of the antibody molecule might decrease during fermentation.

The value for the threshold ratio of course depends among others on the antibody to be purified, the fermentation conditions, the demands of the Health Authorities, and the downstream and formulation procedures used (chromatography steps, filtration etc) and thus has to be defined for each therapeutic antibody production process individually.

Below a defined threshold ratio the relative amount of a certain, for example unwanted variant, is relatively high and the amount of the antibody molecule of interest is relatively low. It has been found that in this case, the gradient elution mode, followed by step elution on the same column, is suitable for purifying the antibody molecule from the variant to such an extent that a drug product can be obtained which is suitable for the market. The step elution only mode, however, is less suitable in this case, due to the lower degree of purification of the antibody molecule from the variant thereof. In the worst case, step elution would not lead to a drug product suitable for the market, if the antibody containing solution to be purified has a ratio of the amount of the antibody molecule to the sum of the amounts of the antibody molecule and a variant thereof below the threshold ratio. See the examples below.

On the other hand, if the ratio of the amount of the antibody molecule to the sum of the amounts of the antibody molecule and the variant thereof in an antibody containing solution to be purified is higher than the threshold ratio, then a step elution only mode during cation exchange chromatography is preferred, since this elution mode, in comparison to gradient elution, followed by step elution, results in higher yields and still a drug product can be obtained which is suitable for the market. In other words, due to the higher quality of the source material applied to the cation exchange column, expressed by the ratio, an elution mode can be chosen which is optimized for yield (step elution).

Since the step elution mode during cation exchange chromatography is favorable with regard to yield, the harvesting conditions can be chosen such that the ratio of the amount of the antibody molecule to the sum of the amounts of the antibody molecule and the variant thereof is always higher than the threshold ratio and step elution would thus always lead to an acceptable drug product.

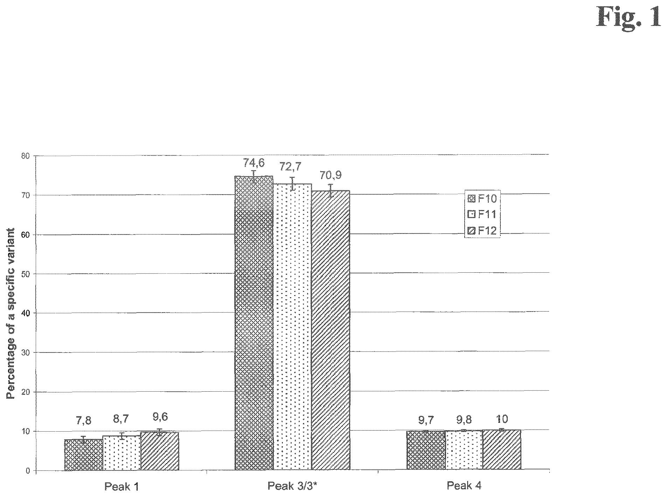

Preferably, the antibody is recovered from the cell culture medium when the ratio of the amount of the antibody molecule to the sum of the amounts of the antibody molecule and the variant thereof ratio is 0-2% higher than the threshold ratio, and most preferred it is very close to or identical to the threshold ratio. Thereby, it is exploited that the fermentation lasts as long as possible for obtaining high absolute amounts of the antibody but not too long in order to avoid an unfavorable ratio of the amount of the antibody molecule to the sum of the amounts of the antibody molecule and the variant thereof. According to our findings after a certain length of fermentation, the percentage of the antibody molecule decreases, whereas the percentages of the variants thereof increase (see Fig. 1 for Herceptin).

In a certain aspect of the current invention, the threshold ratio for determining whether an antibody will be separated by cation exchange chromatography either with gradient, followed by step elution, or with step elution only, is from 50 to 100 %, preferably in the range 60 to 80% and most preferred in the range 65 to 75%. In a further preferred embodiment of the current invention, the threshold ratio is 67.5%.

The antibody according to the current invention is preferably produced by recombinant means. Thus, one aspect of the current invention is a nucleic acid encoding the antibody according to the invention and a further aspect is a cell comprising said nucleic acid encoding an antibody according to the invention. General methods for recombinant production of antibodies are well-known in the state of the art and comprise protein expression in prokaryotic or eukaryotic cells with subsequent isolation of the antibody and usually purification to a pharmaceutically acceptable product (see for example the following reviews: Makrides, S.C., Protein Expr. Purif. 17, 183-202 (1999); Geisse, S., et al., Protein Expr. Purif. 8 (1996) 271-282; Kaufman, R.J., Mol. Biotechnol. 16 (2000) 151-160; Werner, R.G., Drug Res. 48 (1998) 870-880.

For the expression of the antibodies as aforementioned in a host cell, nucleic acids encoding the respective modified light and heavy chains are inserted into expression vectors by standard methods. The hybridoma cells can serve as a source of such DNA and RNA. Once isolated, the DNA may be inserted into expression vectors, which are then transfected into host cells such as CHO cells, NS0 cells, SP2/0 cells, HEK293 cells, PER.C6 cells, yeast, E. Coli cells, or myeloma cells that do not otherwise produce immunoglobulin protein, to obtain the synthesis of recombinant monoclonal antibodies in the host cells. The host cell is cultured under conditions which are suitable for the expression of the heterologous polypeptide and the heterologous polypeptide is isolated from the cells or the culture supernatant.

Generally, the methods and compositions of the invention are useful for the production of recombinant proteins. Recombinant proteins are proteins produced by the process of genetic engineering. Particularly preferred proteins for production according to the methods and compositions of the invention, are protein-based therapeutics, also known as biologics. Preferably, the proteins are secreted as extracellular products.

In one embodiment of the method of the current invention the cell is a recombinant cell clone capable of expressing the heterologous polypeptide.

The method according to the current invention is in principal suitable for the production of any antibody. In one embodiment the immunoglobulins produced with the method according to the invention are recombinant immunoglobulins. In other embodiments the immunoglobulins are humanized immunoglobulins, immunoglobulin fragments, immunoglobulin conjugates or chimeric immunoglobulins.

In another aspect of the current invention the antibody is selected from the group of monoclonal and polyclonal antibodies. In a preferred embodiment the antibody is a monoclonal antibody.

Another aspect of the current invention is the purification of an immunoglobulin of the IgG or IgE class. In one preferred embodiment the antibodies are monoclonal antibodies. In still another embodiment the antibodies produced by the methods according to the current invention are therapeutic or diagnostic antibodies. In one preferred embodiment the antibodies are therapeutic antibodies.

Cells useful in the method according to the invention for the production of a he-terologous polypeptide can in principle be any eukaryotic cells such as e.g. yeast cells or insect cells or prokaryotic cell. However, in one embodiment of the invention the eukaryotic cell is a mammalian cell. Preferably said mammalian cell is a CHO cell line, or a BHK cell line, or a HEK293 cell line, or a human cell line, such as PER.C6®. Furthermore, in one embodiment of the invention the eukaryotic cells are continuous cell lines of animal or human origin, such as e.g. the human cell lines HeLaS3 (Puck, T.T., et al., J. Exp. Meth. 103 (1956) 273-284), (Nadkarni, J.S., et al., Cancer 23 (1969) 64-79), HT1080 (Rasheed, S., et al., Cancer 33 (1974) 1027-1033), or cell lines derived there from.

In a certain aspect of the current invention the cell is cultivated in medium under conditions that the antibody is expressed. The recombinant cell clones can be cultured generally in any desired manner. The nutrients added according to this aspect of the invention comprise essential amino acids, such as e.g. glutamine, or tryptophan, or/and carbohydrates, and optionally non-essential amino acids, vitamins, trace elements, salts, or/and growth factors such as e.g. insulin, and/or peptides, e.g. derived from plants. In one embodiment, the nutrients are added over the entire growth phase (cultivation) of the cells. The cell culture according to the present invention is prepared in a medium suitable for the cultured cell. In one embodiment of the invention, the cultured cell is a CHO cell. Suitable culture conditions for mammalian cells are known (see e.g. Cleveland, W.L., et al., J. Immunol. Methods 56 (1983), 221-234). Moreover, the necessary nutrients and growth factors for the medium, including their amounts, for a particular cell line, can be determined empirically without undue experimentation as described, for example, by Mather (ed.) in: Mammalian cell culture, Plenum Press, NY (1984); Rickwood, D., and Hames, B.D. (eds.), Animal cell culture: A Practical Approach, 2nd ed., Oxford University Press, NY (1992); Barnes and Sato, Cell 22 (1980) 649.

The term "under conditions suitable for the expression" denotes conditions which are used for the cultivation of a cell expressing a polypeptide and which are known to or can easily be determined by a person skilled in the art. It is known to a person skilled in the art that these conditions may vary depending on the type of cell cultivated and type of polypeptide expressed. In general the cell is cultivated at a temperature, e.g. between 20° C and 40°C, and for a period of time sufficient to allow effective production of the conjugate, e.g. from 4 to 28 days.

In one embodiment of the invention, the culture is a suspension culture. Furthermore, in another embodiment the cells are cultured in a medium containing low serum content, such as, e.g., a maximum of 1% (v/v). In a preferred embodiment the culture is a serum-free culture, e.g. in a serum-free, low-protein fermentation medium (see e.g. WO 96/35718) and in a still further preferred environment the medium is protein-fee and/or completely synthetic. Commercially available media such Ham's F10 or F12 (Sigma), Minimal Essential Medium (MEM, Sigma), RPMI-1640 (Sigma), or Dulbecco's Modified Eagle's Medium (DMEM, Sigma), containing appropriate additives are exemplary nutrient solutions. Any of these media may be supplemented as necessary with components as mentioned above.

Cell culture procedures for the large- or small-scale production of antibodies are potentially useful within the context of the present invention. Procedures including, but not limited to, a fluidized bed bioreactor, hollow fiber bioreactor, roller bottle culture, or stirred tank bioreactor system may be used, in the latter two systems, with or without microcarriers. The systems can be operated in one of a batch, a fed-batch, a split-batch, a continuous, or a continuous-perfusion mode. In certain embodiments of the invention, the culture is carried out as a split-batch process with feeding according to requirements of the culture in which a portion of the culture broth is harvested after a growth phase and the remainder of the culture broth remains in the fermenter which is subsequently supplied with fresh medium up to the working volume. The process according to the invention enables the desired antibody to be harvested in very high yields.

According to another aspect of the invention, fed-batch or continuous cell culture conditions are devised to enhance growth of the mammalian cells in the growth phase of the cell culture. In the growth phase cells are grown under conditions and for a period of time that is maximized for growth. Culture conditions, such as temperature, pH, dissolved oxygen ($DO_2$) etc. are those used with the particular host and are known by the skilled person.

According to the present invention, the cell-culture environment during the production phase of the cell culture is controlled. The culture conditions for the antibodies to be produced are defined by the following parameter:

a) Basic medium: amounts and types of nutrient amounts, optional plasma components, growth factors, salts and buffers, nucleosides and bases, protein hydrolyzates, antibiotics and lipids, suitable carriers,

b) Types and amounts of carbohydrate amounts, dissolved oxygen, amount, ammonium amount, pH value, osmolality, temperature, cell density, growth state,

c) Optionally further additives (e.g. plasma components, growth factors such as, e.g., serum components, growth hormones, peptide hydrolyzates, small molecules (like dexamethason, cortisol, iron chelating agents, etc.), inorganic compounds (like selene etc.), and compounds known to have an effect of the glycosylation profile (like butyrate or quinidine, alkanoic acid, or copper, insulin, transferrin, EGF, hormones, salts, inorganic ions, buffers, nucleosides and bases, protein hydrolyzates, antibiotics, lipids, such as, e.g., linoleic acid) are added.

Further additives are for example non-essential compounds stimulating either cell growth and/or enhancing cell survival and/or manipulating the glycosylation profile of a glycosylated antibody in any desired direction.

The polypeptide production phase generally begins at least 3 hours after the beginning of the growth phase, such as at about 12 to about 224 hours, or alternatively at about 120 to 192 hours after the beginning of the growth phase. The production phase can last, e.g., from 4 to 14 days. Alternatively, the production phase may be 18-21 days or even longer, for example up to 28 d. During this phase, cell growth has generally plateaued, e.g., logarithmic cell growth has ended and protein production is primary.

In one aspect of the current, intermittent sampling of the culture medium can be employed, e.g. for determination of specific cell culture parameter (lactate production, antibody amount) or quality check of the antibody produced (determination of the relative amount of active and inactive variants).

In a certain aspect of the current invention, the cells are cultivated in medium under conditions suitable for the expression of specific antibodies and leading to a certain percentage of the antibody relative to a variant thereof. This percentage can vary during fermentation, i.e. could increase or decrease, either predictable (e.g. linear decrease/increase or according to a specific formula) or not predictable. Generally, antibodies produced by host cells are not identical with regards to structure and amino acid sequence, although the nucleic acid encoding the antibodies are identical, but are modified during fermentation and later on by different processes. Common modifications are for example deamidation and glycosylation, which can lead to charge heterogeneity of the antibodies produced in a single fermentation batch. Other modifications leading to charge heterogeneity are incomplete disulfide bond formation, N-terminal pyroglutamine cyclization, C-terminal lysine processing, isomerization, and oxidation, and less common modification of the N-terminal amino acids by maleuric acid and amidation of the C-terminal amino acid.

The harvesting time point represents the time point at which, during fermentation of an antibody, the antibody is harvested, i.e. withdrawn from the culture and either frozen or separated from the medium, for example. This time point is chosen according to the invention on basis of the composition or quality of the antibody containing medium, expressed by the ratio of the amount of the antibody molecule or variant thereof to the sum of the amounts of the antibody molecule and variants thereof. For example the presence or occurrence of a certain variant (acidic, basic, glyco-variant for example) might determine that the fermentation had to be stopped, for example in order to avoid further enrichment of unwanted, for example less active, antibody variant during the further cultivation. But also the relative amount of a certain variant to the antibody molecule of interest, i.e. the ratio of a variant of the antibody molecule and the sum of the amounts of the antibody molecule and a variant thereof, might determine the harvesting time point in order to obtain a desired antibody composition determining a preferred purification scheme.

The ratios determined could then be compared with a (defined) threshold ratio at which, for example, the fermentation must be stopped or had to be continued, such that a certain chromatography protocol can be followed in order to obtain the desired antibody composition. It might for example be the case, that the relative amount of certain unfavorable variants is relatively high, or the relative amount of the antibody molecule of interest is low, in a certain sample, then that purification protocol had to be chosen, which allow the purification of the antibody molecule of interest such that the unfavorable variant is as low as tolerated. On the other hand, the ratio determined in step b) might indicate that the fermentation must be continued further until a desired ratio is obtained. The determination of the ratio can be repeatedly performed but the ratio can also be extrapolated for the further cultivation, see below.

The duration of the fermentation of an antibody producing cell line and the conditions during the downstream procedure are exemplary parameter which here are shown to affect the relative amount of for example the deamidated variant in comparison to the antibody molecule of interest in a sample, see the Examples below.

Assuming that the downstream procedure sets a certain limit for purification of closely related antibody variants, which may only differ in the charge of a single amino acid, then, for example, the level of the antibody molecule in the fermentation medium must not fall below a certain threshold value, such that the downstream procedure still result in a sufficiently pure bulk drug product with high yields. Therefore, the fermentation had to be stopped at a specific time point, e.g. by recovering the antibody from the cell culture medium, when the relative amount of the antibody molecule amount is higher than a defined threshold ratio.

According to a preferred aspect of the current invention, the time point at which the antibody is recovered from the culture medium, is derived from a comparison with a threshold ratio, previously defined.

In a preferred embodiment of the current invention the harvesting time point is determined by estimating the ratio of the amount of the antibody molecule or variant thereof and the sum of the amounts of the antibody molecule and the variant thereof by assuming a linear decrease or increase of the relative amount of the antibody molecule by a certain daily percentage. Specifically in this case a single measurement of the ratio of the antibody molecule or variant thereof to the sum of the amounts of the antibody molecule and a variant thereof by analytical ion exchange chromatography, might be sufficient for determining the time point for recovering the antibody from the culture medium, since the time point can be calculated instead of being derived from further measurements. In a certain embodiment the time point for antibody recovery is calculated by assuming that the amount of the antibody molecule in the medium decreases after a certain length of cultivation by a certain daily percentage.

According to another preferred aspect of the current invention, the sampling and determination of the ratio of the amount of the antibody molecule to the sum of the amounts of the antibody molecule and a variant thereof is performed until the ratio is 0-2 % above a certain threshold ratio, and/or is done by extrapolating the ratio of the amount of the antibody molecule to the sum of the amounts of the antibody molecule and a variant thereof in the medium for the cultivation following the day at which the sample in step iii) has been obtained, by calculating that ratio according to the following formula:

$$R_x = R_0 - C \times (D_x - D_0),$$

wherein

$D_0$ is a cultivation day, at which the sample in step c) is obtained,

$D_x$ is a cultivation day following $D_0$,

$R_x$ is the ratio of the amount of the antibody molecule to the sum of the amount of the antibody molecule and a variant thereof at $D_x$,

$R_0$ is the ratio of the amount of the antibody molecule to sum of the amounts of the antibody molecule and a variant thereof at $D_0$, determined in step iv) and

C is a value in the range 1 %/day and 5 %/day,

The antibody molecule is then recovered from the medium when $R_x$ in the medium is 0-2 % above the threshold ratio.

Preferably, the antibody molecule is recovered from the medium when $R_x$ in the medium is 0-2 % above the threshold ratio, most preferably when the ratio is close to or even identical to the threshold value.

As it is discussed above, the threshold ratio depends among others on the nature of the antibody molecule and on the degree of purity to be obtained in the antibody composition.

In another embodiment of the current invention the amount of the active *her2* variant, corresponding to peak 3/3* in the ion-exchange chromatogram, decreases daily by about 2% after a certain time of cultivation, e.g. after 10d of cultivation (see Fig. 1).

In a certain aspect of the current invention $R_x$ is determined only once, preferably shortly before harvest (e.g. 1-2 d before harvest or at d 11 for Herceptin fermentation for example). If the thus determined $R_x$ is for example 2 % above the threshold ratio, then the cultivation is performed for one further day and the harvest of the antibody thus occurs one day after determination of $R_x$. In the case of Herceptin fermentation $R_x$ at harvest will then be very close to the threshold ratio, since the ratio decreases from d11 to d12 of the fermentation by about 2%, which is known from a number of previous fermentation runs.

If, however, the determined $R_x$ at d 11 is less than 2 % above the threshold ratio, e.g. 1.5 % above the ratio at fermentation day 11 (d11), then the Herceptin fermentation is immediately stopped to assure a sufficient high quality of the bulk drug product.

In a preferred aspect of the current invention, a sample from the cultivation medium comprising the antibody molecule and variant thereof is obtained. In still further embodiments the sampling is performed more than once, e.g. daily, every second d, etc., or even more than once per day. This sampling can start immediately with beginning of the culture or after a certain time, e.g. after 7 or 10d of cultivation. In this sample, the ratio of the amount of the antibody molecule and/or variant thereof and the sum of the amounts of the antibody molecule and a variant thereof, e.g. the percentage of the active antibody variant for example, is determined. Alternatively, other parameter are determined in this sample. The sampling can be performed by methods known by the skilled person in the art. In other aspects of the current invention, also the absolute amount of active antibody can be determined.

The sampling can either be done automatically or manually. In certain embodiments of the invention, the sampling step is performed automatically. The sample volume can range for example from 1 µl to 10 000 µl.

In another embodiment of the current invention, the percentage of the active *her2* antibody variant in the bulk drug product obtainable with the described methods, is ≥ 65% and the percentage of the inactive *her2* variant is ≤ 20%, wherein the active *her2* variant corresponds to peak 3/3* in the ion exchange chromatogram, and the inactive variant in this context corresponds to peak 4 of this chromatogram (see Fig. 1).

In a certain aspect of the current invention, the ratio of the amount of the antibody molecule to the sum of the amounts of the antibody molecule and a variant thereof in the antibody composition produced is from 50-100%, preferably from 60 to 100% and most preferred from 65 to 100%. These values depend in particular from the antibody to be purified.

In a certain aspect of the current invention, the polypeptide of interest preferably is recovered from the culture medium as a secreted polypeptide, although it also may be recovered from host cell lysates when directly expressed without a secretory signal. Harvesting, i.e. recovery of the antibody from the cell culture medium, is performed by methods known by the skilled person in the art, e.g. by separating the cells from the fermentation medium. As a first step, the culture medium or lysate for example is centrifuged to remove particulate cell debris and cells. Depth filtration or other filtration and/or centrifugation steps might follow. Thereafter an antibody containing solution, free of cells and cellular debris, is obtained.

The polypeptide is then purified from contaminants with the following procedures being exemplary of suitable purification procedures: by fractionation on immunoaffinity and ion-exchange columns; ethanol precipitation; reverse phase HPLC; chromatography on silica or on a cation exchange resin such as DEAE; chromatofocusing; SDS-PAGE; ammonium sulfate precipitation; gel filtration using, for example, Sephadex G-75; and protein A Sepharose columns to remove contaminants. A protease inhibitor such as phenyl methyl sulfonyl fluoride (PMSF) or EDTA also may be useful to inhibit proteolytic degradation during purification. One skilled in the art will appreciate that purification methods suitable for the polypeptide of interest may require modification to account for changes in the character of the polypeptide upon expression in recombinant cell culture.

In preferred embodiments of the current invention the purification of the antibody involves affinity chromatography, followed by cation exchange chromatography. In between both chromatography steps further chromatography and/or filtration steps suitable for the purification of antibodies, known by the person of skill in the art, can be included, as well as further chromatography and/or filtration steps after the cation exchange chromatography step and before the Protein A affinity step. It is also possible to reverse the order of the chromatography steps. Prior to the application of a solution to one step (or to a subsequent step) of a purification method, parameters, such as e.g. pH value or conductivity of the solution, have to be adjusted.

In a preferred aspect of the current invention, the medium containing the antibody molecule and a variant thereof is applied to an affinity chromatography column and affinity chromatography is performed. In still certain aspects of the current invention an affinity chromatography step as the foremost purification step is employed for the removal of the bulk of the host cell proteins and culture by-products. The conditions for this step are known to a person of skill in the art. In certain aspects of the current invention, is the affinity column material protein A material, protein G material, metal affinity chromatography material, hydrophobic charge induction chromatography material (HCIC), or hydrophobic interaction chromatography material (HIC, e.g. with phenyl-sepharose, aza-arenophilic resins, or m-aminophenylboronic acid). Preferably the affinity column material is protein A material or metal affinity chromatography material.

In a certain aspect of the current invention, Protein A immobilized on a solid phase is used to purify the antibodies. The solid phase is preferably a column comprising a glass or silica surface for immobilizing the Protein A. Preferably, the solid phase is a controlled pore glass column or a silicic acid column. Sometimes, the column has been coated with a reagent, such as glycerol, in an attempt to prevent nonspecific adherence to the column. The PROSEP A column, commercially available from Bioprocessing Limited, is an example of a Protein A controlled pore glass column which is coated with glycerol. In certain aspects of the current invention, the Protein A material is characterized by a binding capacity higher than 25 mg antibody/ml Protein A material. In still further aspects of the current invention high-flow agarose base matrix, to which Protein A is coupled to the base matrix at the C-terminal cysteine via epoxy activation is employed in the affinity chromatography step. Exemplary materials for the Protein A materials suitable for the methods according to the invention are MabSelect Sure and MabSelect Xtra (both obtainable from GE Healthcare, Lifesciences, Germany).

In a certain aspect of the current invention, the solid phase for Protein A chromatography is equilibrated with a suitable buffer. For example, the equilibration buffer may be TRIS, NaCl, pH 7.1. In a certain aspect of this invention this buffer contains EDTA or another protease inhibitor and/or antibody stabilizer.

In a certain aspect of the current invention, the antibody-containing medium separated from the cells and cell debris, for example by centrifugation and/or filtration or depth filtration, is loaded on the equilibrated solid phase using a loading buffer which may be the same as the equilibration buffer. As the contaminated preparation flows through the solid phase, the protein is adsorbed to the immobilized Protein A and other contaminants such as Chinese Hamster Ovary Proteins (CHOP), when the protein is produced in a CHO cell and DNA bind nonspecifically to the solid phase.

The next step performed afterwards, comprises removing the contaminants bound to the solid phase. In certain aspects of the current invention, the wash buffer includes a hydrophobic electrolyte solvent in a wash step, as for example TMAC and/or TEAC (from about 0.1 to about 1.0 M) of may include a divalent ion or a solvent. Suitable buffers for this purpose include for example TRIS, phosphate, MES, and MOPSO buffers (see the Examples below).

In another aspect of the current invention, the wash buffer comprises arginine at neutral or acidic pH.

Following the wash step mentioned above, the protein of interest is recovered from the column with a suitable elution buffer. The protein may, for example, be eluted from the column using an elution buffer having a low pH, e.g. in the range from about 2 to about 5. Examples of elution buffers for this purpose include citrate or acetate buffers. Alternatively, the elution buffer comprises arginine, divalent salts or solvents.

The eluted protein preparation may be subjected to additional purification steps. Exemplary further purification steps include hydroxylapatite chromatography; dialysis, affinity chromatography using an antibody to capture the protein, hydrophobic interaction chromatography (HIC), hydrophobic charge interaction chromatography (HCIC), ammonium sulphate precipitation, anion or cation exchange chromatography, ethanol precipitation; reverse phase HPLC; chromatography on silica, chromatofocusing and gel filtration.

In a preferred aspect of the current invention, cation exchange chromatography is employed after affinity chromatography. In a certain aspect of the current invention the cation exchange chromatography follows the affinity chromatography step directly, without other chromatography steps in between. In further aspects of the current invention, other chromatography steps and/or purification steps are employed between affinity and cation exchange chromatography.

This cation exchange chromatography step is aimed at reducing not only the amount of unwanted antibody variants, host cell protein (HCP), but also leached protein A, other leached material, , and/or viruses in the solution containing the proteins to be purified.

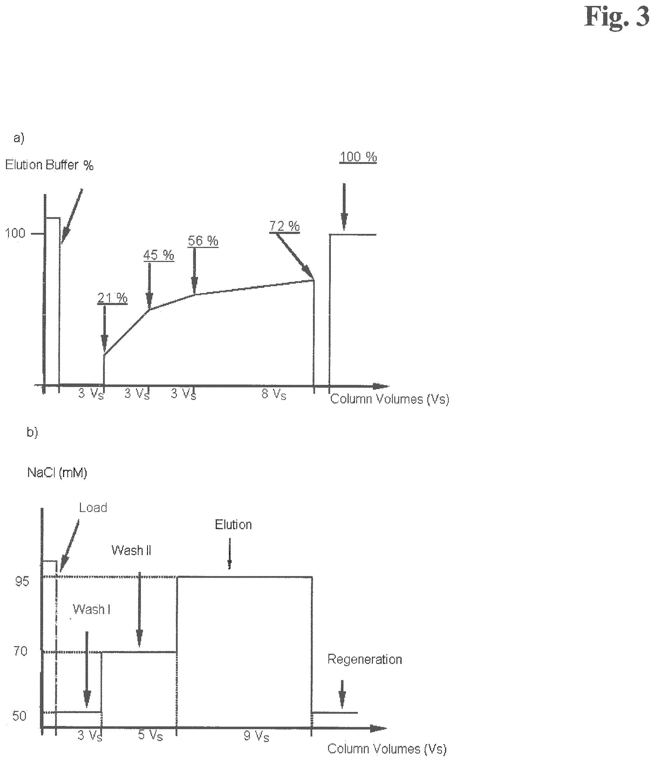

According to a certain aspect of the current invention a cation exchange chromatography step is performed for purifying the antibody. With particular reference to Fig. 3, which shows exemplary buffer profiles which can be used during cation exchange chromatography, the pH and/or conductivity of each buffer is/are increased relative to the preceding buffer. The aqueous solution comprising the polypeptide of interest and contaminant(s) is loaded onto the cation exchange resin using the loading buffer that is at a pH and/or conductivity such that the polypeptide and the contaminant bind to the cation exchange resin.

Exemplary, the conductivity of the loading buffer may be low, e.g. from about 5.2 to about 6.6 mS/cm). An exemplary pH for the loading buffer may be 5.7 mS/cm.

In the step or gradient elution mode according to the current invention, the cation exchange resin can be washed with wash buffers of increasing conductivity and/or pH. In the step elution mode, the first wash buffer could have the same conductivity and/or pH as the loading buffer. In the example below the cation exchange column is washed with wash buffer I (same conductivity and pH as the loading buffer, i.e pH 5.6, $\kappa$ = 5,7$\pm$ 0,5 mS/cm) and then with wash buffer II which has at a second conductivity and/or pH so as to elute most of the contaminant, but not the polypeptide of interest. This may be achieved by increasing the conductivity or pH, or both, of the wash buffer. In certain aspects of the current invention this buffer has a conductivity of 7.0 to 8.5 mS/cm, preferably 7.6 +/- 0.5 mS/cm. The change from wash buffer I to wash buffer II is step-wise in the step elution mode.

Exemplary, wash buffer II had a greater conductivity than that of the loading buffer and wash buffer I. Alternatively, the pH of the wash buffer II may exceed that of the loading buffer and wash buffer I.

In the gradient elution mode, during the washing of the cation exchange column, the pH and/or conductivity is increased continuously, but not necessarily linearly, by washing with a defined percentage of the elution buffer in the load buffer for example (in the examples below, from 21% elution buffer to 72% elution buffer). A multislope gradient is preferred in the current invention.

After the wash steps, either in the gradient or step elution mode, elution is achieved with an elution buffer that has a pH and/or conductivity such that the desired polypeptide no longer binds to the cation exchange resin and thus could be eluted from the column. The pH and/or conductivity of the elution buffer generally exceed(s) the pH and/or conductivity of the loading buffer, the wash buffer I, and the wash buffer II used in the previous steps in the step elution mode. Exemplary, the conductivity of the elution buffer was in the range from about 9.5 to about 11 mS/cm.

The changes in conductivity and/or pH during elution of the antibody is step-wise for elution in the step elution mode and gradual for gradient elution.

In certain aspects of the current invention, a single parameter, either conductivity or pH, is changed for step or gradient elution from the cation exchange column of both the polypeptide and contaminant, while the other parameter (i.e. pH or conductivity, respectively) remains about constant.

In further certain aspects of the current invention, the ion exchange resin is regenerated with a regeneration buffer after elution of the polypeptide, such that the column can be reused.

In a preferred aspect of the current invention, the cation exchange material used for the cation exchange chromatography step is a strong cation exchanger with a sulfopropyl group, like Fast Flow Sepharose FF (GE Healthcare, Lifesciences, Germany). In another aspect of the current invention other suitable cation exchange materials known by the person of skill in the art can be used.

In another aspect of the current invention, the proteins are separated after affinity chromatography employing an anion exchange resin prior to or after the cation exchange chromatography step in the methods of the current invention. The changes in conductivity are generally as described above with respect to a cation exchange resin. However, the direction of change in pH is different for an anion exchange resin. Alternatively, anion exchange chromatography is performed in the flow-through mode.

The antibody composition recovered after the ion exchange chromatography step may be subjected to further purification steps, if necessary, as discussed above. In a certain aspect of the current invention, the proteins are separated after affinity chromatography employing further three or more chromatography steps, for example cation exchange chromatography, anion exchange chromatography and hydrophobic interaction chromatography in any order.

The chromatography methods disclosed here are particularly useful for separating an antibody molecule from at least one contaminant, where the contaminant and the antibody molecule of interest differ only slightly in their isoelectric points, as it is the case for proteins showing charge heterogeneity, e.g. due to deamidation or isomerization. With the methods according to the invention polypeptides and contaminants can be resolved, if their pIs differ by only about 0.05 to about 0.2 pI units. In the Examples below, this method could be used to resolve a *her2* antibody having a pI of 8.87, from a singly-deamidated variant thereof having a pI of 8.79 (cf. Harris et al. R. J. et al. J. Chromatogr. B 752 (2001), 233-245).

In another embodiment of the current invention, the method may be used to resolve an antibody molecule from a glyco-variant thereof, e.g. for resolving a variant of a polypeptide having a different distribution of sialic acid compared to the nonvariant polypeptide.

In another aspect of the current invention, the antibody to be separated is conjugated to one or more heterologous molecules. This heterologous molecule e.g. could increase the serum half-life of the polypeptide (e.g. PEG), a cytotoxic molecule (e.g. a toxin, chemotherapeutic drug, or radioactive isotope etc) or it may be a label (e.g. an enzyme, fluorescent label and/or radionuclide).

In certain aspects of the current invention the antibody containing solution to be purified can be any material containing antibodies containing contaminant and a certain level of the active antibody variant, e.g. samples pre-purified by filtration or chromatographic methods.

In one embodiment of the current invention, the cation exchange chromatography step is performed employing gradient elution, followed by step elution in a single chromatography step.

In a certain aspect of the current invention, the antibody to be purified a monoclonal antibody.

In a further aspect of the invention, the antibody is directed to a tumor antigen (e.g. growth factor receptors and growth factors), selected from the group consisting of EGFR, HER3, HER4, Ep-CAM, CEA, TRAIL, TRAIL-receptor 1, TRAIL-receptor 2, lymphotoxin-beta receptor, CCR4, CD19, CD20, CD22, CD28, CD33, CD40, CD80, CSF-1R, CTLA-4, fibroblast activation protein (FAP), hepsin, melanoma-associated chondroitin sulfate proteoglycan (MCSP), prostate-specific membrane antigen (PSMA), VEGF receptor 1, VEGF receptor 2, IGF1-R, TSLP-R, PDGF-R, TIE-1, TIE-2, TNF-alpha, TNF like weak inducer of apoptosis (TWEAK), IL-1R, preferably EGFR, CEA, CD20, or IGF1-R, and growth factors involved in tumor formation, like VEGF, EGF, PDGF, HGF and angiopoietin.

In another embodiment, the monoclonal antibody is selected from the group of: alemtuzumab, apolizumab, cetuximab, epratuzumab, galiximab, gemtuzumab, ipilimumab, labetuzumab, panitumumab, rituximab, nimotuzumab, mapatumumab, matuzumab and pertuzumab, ING-1, an anti-Ep-CAM antibody being developed by Xoma, preferably trastuzumab, cetuximab, and pertuzumab, more preferably trastuzumab.

In a preferred aspect of the current invention, the antibody to be purified is a monoclonal anti-HER2 antibody, i.e. *her2* antibody, preferably trastuzumab or pertuzumab, more preferably trastuzumab.

In a preferred aspect of the current invention, the bulk drug *her2* product produced by the method according to the invention has a content of active antibody of more than 65% and less than 20% of the inactive antibody, corresponding to peaks 3/3* and peak 4 in the ion exchange chromatogram, respectively (see Fig. 2).

An example for gradient elution, followed by step elution in a single chromatography step is shown in Fig. 3a. In gradient elution or continuous elution, according to the invention, the conductivity and/or pH are continuously increased such that the antibody can be separated from the contaminant. These changes can be linear changes and can be separated by stationary phases. Moreover, the steepness of the linear changes may vary during elution, see for example Fig. 3a. Alternatively, the changes can also be non-linear, fro example exponential. In principal, cation exchange chromatography, either performed in the step elution or gradient elution mode employs similar buffers (see above for the various embodiments). In certain aspects of the current invention, the conductivity of the elution buffer is in the range from about 9.5 to about 11 mS/cm.

In a certain aspect of the current invention, the gradient elution step is followed by a wash step (e.g. same conductivity and/or pH as the loading buffer) and then by step elution in a single chromatography step. In a further aspect, step elution following gradient elution, is performed with 100% of the elution buffer which conductivity is in the range from 9.5 to about 11 mS/cm.

In still further aspects of the current invention, the active variant is eluted from the cation exchange column by raising the conductivity and/or pH step wise after the gradient elution.

In a certain aspect of the current invention the gradient used for elution of the antibody in the cation exchange chromatography step involves the following buffers sequentially: 3.9 column volumes: 21% to 49.4% elution buffer, 3.6 column volumes: 49.4% to 58.8% elution buffer, 7.8 column volumes: 58.8% to 72% elution buffer, 1 column volume: 0% Buffer B, 6.5 column volumes: 100% B.

In the examples below, the active *her2* variant is eluted with a stepwise increase of the conductivity using the buffer profile shown in Fig. 3b.

By adapting the elution mode (gradient or step) in the cation exchange chromatography step to the purity of the source material, i.e. the percentage of the active variant, high yields at a sufficiently high quality can be obtained. In certain aspects of the present invention the elution mode is adapted to the purity with regard to the percentage of the active *her2* variant.

In still other aspects of the current invention, the elution mode is adapted to the percentage of deamidated, acidic or basic variants in the solution to be purified and in certain aspects of the current invention to the percentage of the deamidated and acidic *her2* variants. In further certain aspects of the current invention the elution mode is adapted to the purity with regard to the percentage of certain glyco-variants determined.

It has been found, that with step elution in cation exchange chromatography, higher yields compared to gradient elution followed by step elution can be obtained, but that the separation from contaminating variants is not as good as with the gradient elution mode, followed by step elution. Thus, the former elution type is preferred in case the purity of the protein to be further purified already exceeds a certain threshold value, thereby exploiting the high yields associated with step elution. *Vice versa,* in case the purity of the antibody to be purified further is less than a certain threshold value, step elution is not employed, due to the worse resolution compared to gradient elution, followed by step elution, thereby accepting lower yields. In other words, the purity needed for therapeutic efficacy and safety, can only be obtained with gradient elution, in case the percentage of the most wanted antibody variant, the antibody molecule, in the sample to be purified, is higher than a certain percentage (or the relative amount of the unwanted antibody variant is lower than a certain percentage).

In another preferred aspect of the current invention, the yield, that is the ratio of the amount of the antibody molecule and a variant thereof in the antibody composition produced by the cation exchange chromatography performed by a step wise increase of conductivity and/or pH without a gradual increase of conductivity and/or pH of the buffer applied to the column, to the amount of the antibody molecule and a variant thereof in the eluate of step a2) containing the antibody molecule and a variant thereof loaded onto the cation exchange chromatography, is more than 65%, preferably more than 70% and most preferred more than 75%.

In a further aspect of the current invention, a Protein A affinity chromatography step is performed between the steps of determination of the ratio of the amount of the antibody molecule and the amounts of the antibody molecule and the antibody variant and the cation exchange chromatography step.

The protein recovered after the chromatography steps may be formulated in a pharmaceutically acceptable carrier and is used for various diagnostic, therapeutic or other uses known for such molecules.

A certain aspect of the current invention is directed to a composition comprising a *her2* antibody produced by the methods according to the invention.

The following examples, references, and figures are provided to aid the understanding of the present invention, the true scope of which is set forth in the appended claims. It is understood that modifications can be made in the procedures set forth without departing from the spirit of the invention.

Herceptin®, a *her2* antibody (WO 99/57134), was available in sufficient quantities in our laboratories at the time of the invention and therefore the current invention is exemplified with this immunoglobulin. Likewise the invention is in general practicable with immunoglobulins. This exemplified description is done only by way of example and not by way of limitation of the invention. These examples are provided to aid the understanding of the present invention, the true scope of which is set forth in the appended claims.

**Description of the Figures**

**Figure 1**   Herceptin variant distribution during fermentation. Shown is the percentage of a specific variant, attributable to Peaks 1, 3/3* and 4, respectively, in the ion-exchange chromatogram (see Fig. 3) at fermentation days 10, 11 and 12 (F10, F11 and F12). Shown are average values from 15 fermentation runs and the standard deviation.

**Figure 2**   Analytical ion exchange chromatogram. Trastuzumab separation on a cation exchange column. Peak assignments are given in Table 1.

**Figure 3**   Buffer profiles used for elution in the cation exchange chromatography step: a) gradient elution, b) step elution.

## Example 1

### Change of antibody variant pattern during fermentation

The variant distribution of the Herceptin antibody during fermentation was analyzed for the large scale fermentation of this antibody resulting in the bulk drug product, at days 10, 11 and 12 after start of the culture. Samples were collected at days 10, 11 and 12 and analyzed by analytical ion exchange chromatography for the variant pattern and percentage of variants. The percentage of the variants was calculated from the peak areas in the respective chromatograms obtained. As can be seen from Fig. 1, which summarizes the data obtained from 15 large-scale fermentation runs, there is a clear increase of the variants attributable to peaks 1 and 4 in the ion-exchange chromatogram (compare to Fig. 2 and Table 1), from day 10 to day 12 of the fermentation. Peak 1 corresponds to an acidic, deamidated and less active variant of Herceptin. Peak 4 is composed of a variant with an isomerization of asparagine and/or a Lys450 residue. Moreover, at the same time there is also a decrease of the main peak 3/3*, observed in the samples collected at days 10 to 12, corresponding to the unmodified, most active form of Herceptin. Thus, between fermentation days 10 to 11 there is a clear shift in the variant pattern, i.e. the relative amount of the variants increase whereas the relative amount of the antibody molecule of interest decreases in the same time.

**Table 1:**

| | Assignments for Trastuzumab Cation Exchange Chromatography Peak Fractions | | | | |
|---|---|---|---|---|---|
| Peak | Structural Difference(s) | At Light Chain Asn30 | At Heavy Chain Asn55 | At Heavy Chain Asp102 | Contains NeuAc or Deamidated HC[a] |
| a | Deamidated (to Asp) at Asn30 of both light chains | **Asp/Asp** | Asn/Asn | Asp/Asp | No |
| b | Deamidated (to isoAsp) at Asn55 in one heavy chain | Asn/Asn | Asn/**isoAsp** | Asp/Asp | No |
| 1* | Deamidated (to Asp) at Asn30 of one light chain, acidic form | Asn/**Asp** | Asn/Asn | Asp/Asp | **Yes** |
| 1 | Deamidated (to Asp) at Asn30 of one light chain | Asn/**Asp** | Asn/Asn | Asp/Asp | No |
| 2 | Deamidated (to Asp) at Asn30 of one light chain, and isomerized (to isoAsp) at Asp102 of one heavy chain | Asn/**Asp** | Asn/Asn | Asp/**isoAsp** | No |
| 3* | Main peak, acidic form, or peak 1 form **with one Lys450 residue** | Asn/Asn<br>Asn/**Asp** | Asn/Asn<br>Asn/Asn | Asp/Asp<br>Asp/Asp | **Yes**<br>No |
| 3 | Main peak | Asn/Asn | Asn/Asn | Asp/Asp | No |
| 4 | Isomerized (to isoAsp) at Asp102 of one heavy chain, and/or main peak form **with one Lys450 residue** | Asn/Asn<br>Asn/Asn | Asn/Asn<br>Asn/Asn | Asp/**isoAsp**<br>Asp/Asp | No<br>No |
| c | Succinimide (Asu) at Asp102 position of one heavy chain | Asn/Asn | Asn/Asn | Asp/**Asu** | No |
| Note: Differences from the main peak form are shown in boldface type.<br>[a] Refers to presence of N-acetylneuraminic acid (NeuAc), deamidation in heavy chain at Asn387, or deamidation at heavy chain Asn392. | | | | | |

## Example 2

**Purification of *her2* antibodies with Protein A affinity chromatography and determination of the percentage of active *her2* variants**

**Recombinant DNA techniques:**

Standard methods were used to manipulate DNA as described in Sambrook, J., et al., Molecular Cloning: A Laboratory Manual, Second Edition, Cold Spring Harbor Laboratory Press, Cold Spring Harbor, N.Y., (1989). The molecular biological reagents were used according to the manufacturer's instructions.

**Protein determination:**

The protein amount of each chromatography fraction was determined by spectrophotometric scans of each sample. The results were used to calculate product recovery yields. The extinction coefficient for *her2* is 1.45. Calculations used to derive the results are:

Protein amount (mg/ml) = 280 nm/1.45 x Dilution factor

$$\text{Protein Mass (mg) in each Fraction} = \text{Protein Amount (mg/ml)} \times \text{Fraction Volume (ml)}$$

$$\text{Yield (\%)} = \text{Fraction Mass (mg)} / \text{Total Mass (mg)} \times 100$$

**Host cell protein determination:**

The walls of the wells of a micro titer plate are coated with a mixture of serum albumin and Streptavidin. A goat derived polyclonal antibody against HCP is bound to the walls of the wells of the micro titer plate. After a washing step different wells of the micro titer plate are incubated with a HCP calibration sequence of different amounts and sample solution. After the incubation not bound sample material is removed by washing with buffer solution. For the detection the wells are incubated with an antibody peroxidase conjugate to detect bound host cell protein. The fixed peroxidase activity is detected by incubation with ABTS and detection at 405 nm.

**DNA determination:**

The DNA content in the samples was determined via quantitative PCR according to known procedures.

**Protein A affinity chromatography:**

The following *her2* antibody material resulting from two different fermentations was used:

F1:  *her2* A  <*her2*> = 0,907 mg/mL
F2:  *her2* B  <*her2*> = 1,739 mg/mL

The supernatant resulting from fermentation 2, Material F2, was stored for 12 h at alkaline conditions (pH 9) for generating artificially deamidated antibody material, thus antibody of low quality and with a lower percentage of the active isomer compared to the antibody of F1. The percentage decrease of the 3/3* peak area, corresponding to the amount of active isomer, is visible in the ion exchange chromatogram (see also Table 2). In the following this sample is denoted F2'.

F 2':  *her2* B  <*Her2*> = 1,739 mg/ml

The solution containing *her2* antibody, i.e either F1, F2 and F2', was applied in a first step to a Protein A affinity column.

The chromatographic conditions were as follows:

Resin:      MabSelect SuRe (GE Healthcare, Life Sciences, Germany)

(continued)

| | |
|---|---|
| Equilibration: | 25 mM Tris, 25 mM NaCl, 5 mM EDTA pH; 7,1 |
| Wash step I: | 25 mM Tris; 25 mM NaCl; 5 mM EDTA, , pH 7.1 |
| Wash step II: | 25 mM NaCl; 500 mM TMAC; 5 mM EDTA; pH 5,0 |
| Wash step III: | 25 mM Tris; 25 mM NaCl; 5 mM EDTA pH; 7,1 ± 0,5 |
| Elution: | 25 mM Citrate; pH 2,8 ± 0,4 |

**Analytical ion exchange chromatography:**

Determination of the relative content of the active and inactive *her2* antibody variants was performed via analytical ion exchange HPLC.

Chromatographic conditions:

| | |
|---|---|
| Resin: | Dionex ProPac™ WCX-10 Analytical, 4x 250 mm, Dionex 54993 (weak cation exchange chromatography) |
| Flow rate: | 0.8 ml/min |
| Loading: | 50 μl or 50 μg |
| Pressure: | max 210 bar |
| Buffer A: | 10 mM Na-phosphate, pH 7.5 |
| Buffer B: | 0.1 M NaCl in buffer A |
| Temperature: | Room temperature |

Equilibration, gradient elution and regeneration are performed according to the following scheme:

| Time (min) | Buffer A | Buffer B |
|---|---|---|
| 0 | 85 | 15 |
| 30 | 45 | 55 |
| 35 | 45 | 55 |
| 36 | 0 | 100 |
| 44 | 0 | 100 |
| 45 | 85 | 15 |
| 55 | 85 | 15 |

The percentage of the active form of the *her2* antibody was calculated by determining the relative peak areas of peaks 3 and 3* (denoted 3/3* in the following tables), wherein 3* represents a small shoulder of peak 3. The percentage of the less active *her2* variants were calculated by determining the relative peak areas of peaks 1 and 4. In peak 1, a deamidated, acidic *her2* variant (Asn to Asp at position 30 in one light chain) can be found and in peak 4 a *her2* variant characterized by an iso-aspartate at position 102 in one heavy chain (see Fig. 2 and Table 1).

Table 2 summarizes the quality of the Protein A eluates obtained from the F1, F2 and F2' samples which are used for cation exchange chromatography described below in Example 3. It can be seen that the IEC Peak 3/3*, expressed as relative area under the peak in the analytical ion exchange chromatogram, representing the main form of the *her2* antibody, decreases in the sample exposed to alkaline conditions relative to the sample prior to exposure (54.3% versus 58%) and that, *vice versa,* the percentage of the *her2* variant in Peak 1, i.e. the acidic, deamidated *her2* variant is increased (from 9.7% to 13.3%) after exposure to alkaline conditions.

**Table 2:**

| *Her2* variant pattern after Protein A chromatography of unmodified *her2* samples (F1 and F2) and a *her2* sample exposed to alkaline conditions (F2'). | | | |
|---|---|---|---|
| Sample | **F1** | **F2** | **F2'** |
| SEC [rel. Area] | 98,8 | 98,7 | 98,8 |

(continued)

| Her2 variant pattern after Protein A chromatography of unmodified her2 samples (F1 and F2) and a her2 sample exposed to alkaline conditions (F2'). | | | |
|---|---|---|---|
| Sample | **F1** | **F2** | **F2'** |
| IEC Peak 1 [rel. Area] | 9,7 | 9,8 | 13,3 |
| IEC Peak 3/3* [rel. Area] | 58 | 58,3 | 54,3 |
| IEC Peak 4 [rel. Area] | 17,1 | 18,9 | 16,6 |
| DNA [pgDNA/mg] | 4,5 | 2 | <2,0 |
| HCP [ppm] | 4376 | **1452** | **946** |
| *SEC: Monomer Content, HCP: host cell protein* | | | |

### Example 3

**Purification of affinity purified her2 antibodies with cation exchange chromatography using different elution modes**

Following Protein A chromatography, cation exchange chromatography was performed to further separate the desired her2 antibodies. Prior to cation exchange chromatography the pH of the Protein A eluates was adjusted to 5,5 with 1 M TRIS. Each sample (Protein A eluates of F1, F2 and F2') was purified by cation exchange chromatography on SP Sepharose FF (GE Healthcare) using either gradient, followed by step elution or step elution only, respectively, resulting in six experiments. Each of the resulting chromatograms was analyzed with regard to the monomer content (SEC), variant pattern, in particular the percentages of the active and deamidated variants, DNA and HCP decrease.
The chromatographic conditions were as follows:

| | |
|---|---|
| Resin: | SP Sepharose FF, GE Healthcare |
| Column length: | 35 cm |
| Loading: | conditioned Protein A pool |

**Buffers used for gradient elution, followed by step elution:**

Equilibration buffer and Wash buffer : 30 mM MES, 45 mM NaCl, pH 5.6 $\pm$ 0.05; $\kappa$ = 5.7 $\pm$ 0.5 mS/cm Elution buffer: 30 mM MES, 95 mM NaCl, pH 5,6 $\pm$ 0,05; $\kappa$ = 10.35 $\pm$ 0,65 mS/cm
The gradient used for elution is shown in Fig. 3a.

**Buffers used for step elution:**

Equilibration and Wash buffer I: 25 mM MES, 50 mM NaCl, 5.6 $\pm$ 0,1; $\kappa$ = 5.7$\pm$ 0,5 mS/cm Wash buffer II: 25 mM MES, 70 mM NaCl, pH 5.6$\pm$ 0,1; $\kappa$ = 7.6 $\pm$ 0,5 mS/cm
Elution buffer: 25 mM MES; 95 mM NaCl; pH 5.6 $\pm$ 0,1; $\kappa$ = 10.0 $\pm$ 0.5 mS/cm
The buffer profile used for step elution is shown in Fig. 3b.
The following tables 3 and 4 show the variant distribution, purity and yield of her2 antibodies obtained from the F1 (Table 3) and F2' (Table 4) samples after Protein A affinity and cation exchange chromatography, performed with a step elution only or with gradient elution, followed by step elution.

**Table 3:**

Purity and yield of her2 antibody obtained from the F1 sample

| F1 | | Protein A Eluate | Step Elution only | Gradient Elution, followed by step elution |
|---|---|---|---|---|
| Monomer content (SEC) [% rel. Area] | | 98.8 | 99.7 | 99.6 |
| Variants (IEC) | Peak 1 | 9.7 | 10.4 | 6.9 |
| [% rel. Area] | Peak 3/3* | 58 | 60,9 | 65 |

(continued)

Purity and yield of *her2* antibody obtained from the F1 sample

| F1 | Protein A Eluate | Step Elution only | Gradient Elution, followed by step elution |
|---|---|---|---|
| Peak 4 | 17.1 | 12.7 | 19.8 |
| DNA-content [pg DNA/mg MAK] | 4.5 | 5.06 | 11.43 |
| HCP-content [ppm] | 4376 | 544 | 537 |
| Yield [%] | - | 82 | 44 |
| Antibody amount (mg) | - | 903 | 481 |

**Table 4:**

Purity and yield of *her* antibody obtained from the F2' sample

| F2' | | Protein Eluate | A Step Elution | Gradient Elution, followed by step Elution |
|---|---|---|---|---|
| Monomer content (SEC) [% rel. Area] | | 98.8 | 98.8 | 98,9 |
| Variants (IEC) | Peak 1 | 13.3 | 13,6 | 7,8 |
| [% rel. Area] | Peak 3/3* | 54.3 | 56.3 | 62.3 |
| | Peak 4 | 16.6 | 14 | 24,4 |
| DNA-content [pg DNA/mg MAK] | | < 2.0 | <0.5 | < 2,0 |
| HCP-content [ppm] | | 946 | 327 | 88 |
| Yield [%] | | - | 72 | 46 |
| Antibody amount (mg) | | - | 789 | 502 |

As can be seen from the tables 3 and 4, gradient elution, followed by step elution, results in a higher percentage (4% - 6%) of the most active antibody (3/3* peak) in comparison to step elution only. Gradient and step elutions are equally suited for decreasing the amount of contaminating host cell proteins (HCP). However, the yield is markedly higher with step in comparison to gradient elution (about factor 2). In part this is due to a loss of antibody during the wash gradient in the gradient elution mode. Thus, depending on which parameter needs improvement, either yield of purity, gradient or step elution might be optimal for purification of antibodies.

Further, in case the Protein A eluate already contains a relative high content of the most active antibody variant, the preferred kind of elution in the cation exchange chromatography step is step elution, since a sufficiently pure antibody with a much higher yield compared to gradient elution, followed by step elution, can be obtained. The markedly higher yield corresponds to higher an absolute antibody amount produced which directly results in higher antibody production rates.

However, in case the most active antibody (3/3*) constitutes only less than a certain percentage in the Protein A eluate, gradient elution, followed by step elution, is preferred, since only with this kind of elution a sufficiently active *her2* antibody can be obtained. The lower yields obtained with this method, then have to be accepted. The decrease of contaminating DNA and HCP is similar for both elution modes.

In another aspect of the current invention analytical ion exchange chromatography of an aliquot of a sample comprising a polypeptide is used for determination of a subsequent polypeptide purification scheme of said sample. The polypeptide is preferably an antibody, more preferred a monoclonal antibody and most preferred is a *her2* antibody. The sample is preferably a sample obtained from a cell culture, free of cells and/or cellular debris or is a sample obtained during chromatography for purification of a polypeptide. Preferably the analytical ion exchange chromatography is aimed at resolving antibody variants. Preferably the analytical ion exchange chromatography is a cation exchange chromatography. Preferably, the use of analytical ion exchange chromatography determines a protocol used for the cation exchange chromatography step. Most preferred, in the subsequent purification scheme an antibody is eluted from a cation exchange column

i) with a gradual increase of conductivity and/or pH of the elution buffer applied to the cation exchange column, and

ii) second, by a step wise increase of conductivity and/or pH of the elution buffer applied to the cation exchange column, if the ratio of the amount of the antibody molecule to the sum of the amounts of the antibody molecule and a variant thereof is below a threshold ratio, or

with a step wise increase of conductivity and/or pH without a gradual increase of conductivity and/or pH of the elution buffer applied to the cation exchange column, if the ratio of the amount of the antibody molecule to the sum of the amounts of the antibody molecule and a variant thereof is above a threshold ratio.

The ratio of the amount of the antibody molecule to the sum of the amounts of the antibody molecule and a variant thereof are preferably determined from the peak areas in the analytical ion exchange chromatograms.

The threshold ratio is determined on basis of the purity to be obtained and is among others dependent on the antibody itself and the purification protocol used.

The present invention also relates to the following items:

1. Method for producing an antibody composition comprising an antibody molecule and a variant thereof, comprising the following steps:

a) providing a sample comprising the antibody molecule and a variant thereof,

b) determining the ratio of the amount of the antibody molecule to the sum of the amounts of the antibody molecule and a variant thereof in said sample by an analytical method performed on an aliquot of said sample,

and step c) comprises:

a1) optionally filtrating and/or centrifuging said sample of step a),

a2) optionally performing an affinity chromatography by applying said further filtrated and/or centrifuged sample of step a1) or said sample of step a), on an affinity chromatography column, optionally washing the column and eluting the antibody from the column,

a3) performing a cation exchange chromatography by loading the sample of step a), a1) or eluate of step a2) containing the antibody, optionally after adjusting the pH and/or conductivity, onto a cation exchange chromatography column and optionally washing the column,

a4) eluting the antibody from the cation exchange chromatography column

i) by a gradual increase of conductivity and/or pH of the buffer applied to the cation exchange column, and

ii) second, by a step wise increase of conductivity and/or pH of the elution buffer applied to the cation exchange column,
if the ratio of the amount of the antibody molecule to the sum of the amounts of the antibody molecule and a variant thereof determined in step b) is below a threshold ratio selected from the range of 65 to 75 %.
or
with a step wise increase of conductivity and/or pH without a gradual increase of conductivity and/or pH of the elution buffer applied to the column, if the ratio of the amount of the antibody molecule to the sum of the amounts of the antibody molecule and a variant thereof determined in step a1) is above a threshold ratio selected from the range of 65 to 75%,

thereby producing an antibody composition comprising the antibody molecule and a variant thereof.

2. Method according to item 1, wherein the variant of the antibody molecule is an acidic or basic variant.

3. Method according to item 2, wherein the pI of the antibody molecule differs by 0.1 to 0.5 pI units from the pI of the variant.

4. Method according to any of items 1-3, wherein in step b) the analytical method is ion-exchange chromatography, ELISA, or MALDI-TOF analysis.

5. Method according to any of items 1-4, wherein said sample in step a) is an eluate of an affinity chromatography step or is a cell culture medium comprising an antibody and a variant thereof free from cells and cellular debris.

6. Method according to any of the preceding items, wherein step a) of providing a sample comprises

i) providing a cell containing a nucleic acid molecule comprising a nucleic acid sequence encoding said antibody molecule,

ii) cultivating said cell in a medium for 4-28 days,

iii) obtaining a sample from the medium,

thereby providing the sample comprising the medium, the cell, the antibody molecule and a variant thereof.

7. Method according to item 6, wherein step b) of item 1 consists of

iv) determining the ratio of the amount of the antibody molecule to the sum of the amounts of the antibody molecule and a variant thereof in an aliquot of the sample obtained from the medium in step ii) in item 6, by analytical ion exchange chromatography,

v) optionally repeating steps iii) and iv) until the ratio determined in step iv), is 0-2 % above a threshold ratio selected from the range of 65 % to 75%, and/or extrapolating the ratio of the amount of the antibody molecule to the sum of the amounts of the antibody molecule and a variant thereof in the medium for a specific cultivation day $D_x$, by calculating that ratio according to the following formula:

$$R_x = R_0 - C \times (D_x - D_0),$$

wherein

$D_0$ is a cultivation day, at which the sample in step c) is obtained,

$D_x$ is a cultivation day following $D_0$,

$R_x$ is the ratio of the amount of the antibody molecule to the sum of the amount of the antibody molecule and a variant thereof at $D_x$,

$R_0$ is the ratio of the amount of the antibody molecule to the sum of the amounts of the antibody molecule and a variant thereof at $D_0$, determined in step iv) and

C is a value in the range 1 %/day and 5 %/day, and corresponds to the daily percentage alteration of the ratio of the amount of the antibody molecule to the sum of the amounts of the antibody molecule and a variant

vi) recovering the antibody molecule from the medium when $R_x$ in the medium is 0-2 % above the threshold ratio selected from the range of 65 to 75%,
and step a3) in item 1 comprises

vii) performing a cation exchange chromatography by loading the sample of step vi) containing the antibody, optionally after adjusting the pH and/or conductivity, onto a cation exchange chromatography column and optionally washing the column,

viii) eluting the antibody from the cation exchange chromatography column with a step wise increase of conductivity and/or pH without a gradual increase of conductivity and/or pH of the elution buffer applied to the column,

thereby producing an antibody composition comprising the antibody molecule and a variant thereof.

8. Method according to any of the preceding items, wherein the threshold ratio is 67.5 %.

9. Method according to any of items 1-8, wherein the ratio of the amount of the antibody molecule to the sum of the amounts of the antibody molecule and a variant thereof in the antibody composition produced is from 50 to 100 %, preferably in the range 60 to 100 % and most preferred in the range 65 to 100 %.

10. Method according to any of items 1-9, wherein the yield, that is the ratio of the amount of the antibody molecule and a variant thereof in the antibody composition produced by the cation exchange chromatography performed by a step wise increase of conductivity and/or pH without a gradual increase of conductivity and/or pH of the buffer applied to the column, to the amount of the antibody molecule and a variant thereof in the eluate of step a4) containing the antibody molecule and a variant thereof loaded onto the cation exchange chromatography, is more than 65%, preferably more than 70% and most preferred more than 75%.

11. Method according to any one of items 1-10, characterized in that, if an affinity chromatography step is included, the affinity chromatography is a Protein A, or a Protein G, or metal affinity chromatography.

12. Method according to any of items 1-11, wherein the antibody is a monoclonal antibody.

13. Method according to item 12, wherein the monoclonal antibody is a *her2* antibody.

14. Method according to item 13, wherein the antibody is Trastuzumab.

15. Use of analytical ion exchange chromatography for analyzing a sample comprising an antibody molecule and a variant thereof for determination of a subsequent polypeptide purification scheme of said sample.

16. Use according to item 15, wherein in the subsequent purification scheme an antibody is eluted from a cation exchange column

i) with a gradual increase of conductivity and/or pH of the elution buffer applied to the cation exchange column, and
ii) second, by a step wise increase of conductivity and/or pH of the elution buffer applied to the cation exchange column, if the ratio of the amount of the antibody molecule to the sum of the amounts of the antibody molecule and a variant thereof is below a threshold ratio, or
with a step wise increase of conductivity and/or pH without a gradual increase of conductivity and/or pH of the elution buffer applied to the cation exchange column, if the ratio of the amount of the antibody molecule to the sum of the amounts of the antibody molecule and a variant thereof is above a threshold ratio.

17. Use according to any of items 16 and 17, wherein the antibody is a *her2* antibody.

## Claims

1. Method for producing a trastuzumab composition comprising trastuzumab and a less active variant thereof, comprising the following steps:

a) providing a sample comprising the trastuzumab molecule and a less active variant thereof,
b) determining the ratio of the amount of the trastuzumab molecule to the sum of the amounts of the trastuzumab molecule and the variants thereof in said sample by an analytical method performed on an aliquot of said sample,
c) optionally filtrating and/or centrifuging said sample of step a),
d) performing an affinity chromatography by applying said further filtrated and/or centrifuged sample of step c) or said sample of step a), on an affinity chromatography column,
e) performing a cation exchange chromatography by

e1) loading the eluate of step d) containing trastuzumab, using a loading buffer having a conductivity of 5.2 to 6.6 mS/cm, onto a cation exchange chromatography column,
e2) step-wise washing of the column comprising (i) washing with a first wash buffer which has the same conductivity as the loading buffer; and (ii) washing with a second wash buffer which has a conductivity of 7.0 to 8.5 mS/cm, and
e3) eluting the trastuzumab molecule from the cation exchange chromatography column with an elution buffer which has a conductivity of 9.5 to 11 mS/cm;
if the ratio of the amount of the trastuzumab molecule to the sum of the amounts of the trastuzumab molecule and the variants thereof determined in step b) is above a threshold ratio of 67.5 %,

thereby producing a purified trastuzumab composition comprising the antibody molecule and a less active variant thereof.

2. Method according to claim 1, wherein the pI of the trastuzumab molecule differs by 0.1 to 0.5 pI units from the pI of the variant.

3. Method according to any of claims 1-2, wherein in step b) the analytical method is ionexchange chromatography, ELISA, or MALDI-TOF analysis.

4. Method according to any of claims 1-3, wherein said sample in step a) is a cell culture medium comprising a trastuzumab and a variant thereof free from cells and cellular debris.

5. Method according to any of the preceding claims, wherein step a) of providing a sample comprises

i) providing a cell containing a nucleic acid molecule comprising a nucleic acid sequence encoding said trastuzumab molecule,
ii) cultivating said cell in a medium for 4-28 days,
iii) obtaining a sample from the medium,

thereby providing the sample comprising the medium, the cell, the trastuzumab molecule and a variant thereof.

6. Method according to any one of claims 1-5, wherein the affinity chromatography is a Protein A, or a Protein G, or metal affinity chromatography.

Fig. 1

**Fig. 2**

**Fig. 3**

a)

Elution Buffer %

b)

NaCl (mM)

Europäisches
Patentamt

European
Patent Office

Office européen
des brevets

# EUROPEAN SEARCH REPORT

Application Number

EP 18 16 1305

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 308 455 A2 (GENENTECH INC [US]) 7 May 2003 (2003-05-07) <br> * figures 7A, 7B, 3 * <br> * paragraphs [0097], [0098], [0107] * <br> * paragraph [0055]; figure 5 * <br> * page 14, line 23 * <br> * table 1 * <br> * page 16, lines 26-27, 20-22 * <br> * page 16, lines 28-33 * <br> ----- | 1-6 | INV. <br> C07K16/32 <br> C07K1/18 |
| T | WURM F M: "Production of recombinant protein therapeutics in cultivated mammalian cells", NATURE BIOTECHNOLOGY, GALE GROUP INC, US, vol. 22, no. 11, 1 November 2004 (2004-11-01), pages 1393-1398, XP002667427, ISSN: 1087-0156, DOI: DOI:10.1038/NBT1026 [retrieved on 2004-11-04] * the whole document * <br> ----- | 5 | |
| T | ROBINSON D K ET AL: "Characterization of a recombinant antibody produced in the course of a high yield fed-batch process", BIOTECHNOLOGY AND BIOENGINEERING, WILEY, US, vol. 44, no. 6, 5 September 1994 (1994-09-05), pages 727-735, XP002559794, ISSN: 0006-3592, DOI: 10.1002/BIT.260440609 [retrieved on 2004-02-19] * the whole document * <br> ----- | 1-6 | TECHNICAL FIELDS SEARCHED (IPC) <br><br> C07K <br> A61K |

The present search report has been drawn up for all claims

| Place of search | Date of completion of the search | Examiner |
|---|---|---|
| The Hague | 23 July 2018 | Bumb, Peter |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................

& : member of the same patent family, corresponding document

EPO FORM 1503 03.82 (P04C01)

**EP 3 404 044 A1**

## ANNEX TO THE EUROPEAN SEARCH REPORT
## ON EUROPEAN PATENT APPLICATION NO.

EP 18 16 1305

This annex lists the patent family members relating to the patent documents cited in the above-mentioned European search report.
The members are as contained in the European Patent Office EDP file on
The European Patent Office is in no way liable for these particulars which are merely given for the purpose of information.

23-07-2018

| Patent document cited in search report | | Publication date | Patent family member(s) | | Publication date |
|---|---|---|---|---|---|
| EP 1308455 | A2 | 07-05-2003 | DK | 1308455 T3 | 31-07-2006 |
| | | | EP | 1308455 A2 | 07-05-2003 |
| | | | EP | 1308456 A2 | 07-05-2003 |
| | | | SI | 1308456 T1 | 29-02-2008 |

For more details about this annex : see Official Journal of the European Patent Office, No. 12/82

EPO FORM P0459

34

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- WO 2006084111 A
- US 5821337 A
- EP 1075488 B1
- EP 1308455 B9
- WO 2004024866 A
- WO 9635718 A
- WO 9957134 A

## Non-patent literature cited in the description

- **AHRER, K. ; JUNGBAUER, A.** *J. Chromatogr.,* 2006, vol. B 841, 110-122
- **ASWALD, D.W. et al.** *J. Pharmaceut. Biomed. Anal.,* 2000, vol. 21, 1129-1136
- **LIU, H. et al.** *J. Pharmac. Sciences,* 2008, vol. 97, 2426-2447
- **BECK, A. et al.** *Curr. Pharm. Biotechnol.,* 2008, vol. 9, 482-501
- **TSAI, P.K. et al.** *Pharmac. Res.,* 1993, vol. 10, 1580-1586
- **MOORHOUSE, K.G. et al.** *J. Pharmac. Biomed. Anal.,* 1997, vol. 16, 593-603
- **KALTENBRUNNER, O. et al.** *J. Chrom,* 1993, vol. 639, 41-49
- **ROBINSON, D,K. et al.** *Biotech. and Bioeng,* 1994, vol. 44, 727-735
- **WEITZHANDLER, M.** *Proteomics,* 2001, vol. 1, 179-185
- **HUDZIAK, R.M. et al.** *Mol. Cell. Biol,* 1989, vol. 9, 1165-1172
- **BASELGA, J. et al.** *J. Clin. Oncol.,* 1996, vol. 14, 737-744
- **HARRIS R. J. et al.** *J. Chromatogr. B,* 2001, vol. 752, 233-245
- **SAMBROOK ; FRITSCH ; MANIATIS.** Molecular Cloning: A Laboratory Manual. Cold Spring Harbor Laboratory Press, 1989
- DNA Cloning - A Practical Approach. IRL Press Limited, 1985, vol. I and II
- Oligonucleotide Synthesis - A Practical Approach. IRL Press Limited, 1984
- Nucleic acid hybridisation. IRL Press Limited, 1984
- Animal cell culture - a practical approach. IRL Press Limited, 1986
- **PERBAL, B.** A practical guide to molecular cloning. Wiley Interscience, 1984
- Methods in Enzymology. Academic Press, Inc,
- Gene transfer vectors for mammalian cells. Cold Spring Harbor Laboratory, 1987
- Methods in Enzymology. vol. 154-155
- Immunochemical methods in cell and molecular biology. Academic Press, 1987
- **SCOPES, R.K.** Protein Purification - Principles and Practice. Springer-Verlag, 1987
- Handbook of Experimental Immunology. Blackwell Scientific Publications, 1986
- Part A: Fundamentals and Techniques. Chromatography. Elsevier Science Publishing Company, 1992
- Advanced Chromatographic and Electromigration Methods in Biosciences. Elsevier Science BV, 1998
- **POOLE, C. F. ; POOLE, S. K.** Chromatography Today. Elsevier Science Publishing Company, 1991
- **SCOPES, R.K.** Protein Purification: Principles and Practice. 1987
- Molecular Cloning: A Laboratory Manual. Cold Spring Harbor Laboratory Press, 1989
- Current Protocols in Molecular Biology. John Wiley & Sons, Inc,
- Chromatographical Techniques in the Downstream Processing of (Recombinant) Proteins, Methods in Biotechnology, Vol. 24: Animal Cell Biotechnology. **FREITAG, R.** Methods and Protocols. Humana Press Inc, 2007, vol. 24, 421-453
- **VIJAYALAKSHMI, M.A.** *Appl. Biochem. Biotech.,* 1998, vol. 75, 93-102
- **LUCKOW, V.A. et al.** *Bio/Technology,* 1988, vol. 6, 47-55
- **MILLER, D.W. et al.** Genetic Engineering. Plenum Publishing, 1986, vol. 8, 277-298
- **MAEDA, S. et al.** *Nature,* 1985, vol. 315, 592-594
- **MARTIN, C.R. et al.** *Analytical Chemistry-News & Features,* 1998, 322A-327A
- **SEMBA et al.** *PNAS (USA),* 1985, vol. 82, 6497-6501
- **YAMAMOTO et al.** *Nature,* 1986, vol. 319, 230-234

- **AHRER ; JUNGBAUER.** *J. of Chromatog,* 2006, vol. 841, 110-122
- **HARRIS, R. J.** *J. Chromatogr,* 2001, vol. B 752, 233-245
- **MAKRIDES, S.C.** *Protein Expr. Purif.,* 1999, vol. 17, 183-202
- **GEISSE, S. et al.** *Protein Expr. Purif.,* 1996, vol. 8, 271-282
- **KAUFMAN, R.J.** *Mol. Biotechnol.,* 2000, vol. 16, 151-160
- **WERNER, R.G.** *Drug Res.,* 1998, vol. 48, 870-880
- **PUCK, T.T. et al.** *J. Exp. Meth.,* 1956, vol. 103, 273-284
- **NADKARNI, J.S. et al.** *Cancer,* 1969, vol. 23, 64-79
- **RASHEED, S. et al.** *Cancer,* 1974, vol. 33, 1027-1033
- **CLEVELAND, W.L. et al.** *J. Immunol. Methods,* 1983, vol. 56, 221-234
- Mammalian cell culture. Plenum Press, 1984
- Animal cell culture: A Practical Approach. Oxford University Press, 1992
- **BARNES ; SATO.** *Cell,* 1980, vol. 22, 649
- **HARRIS et al.** *J. Chromatogr.,* 2001, vol. B 752, 233-245
- **SAMBROOK, J. et al.** Molecular Cloning: A Laboratory Manual. Cold Spring Harbor Laboratory Press, 1989